# EUROPEAN PATENT APPLICATION

(11) **EP 4 349 865 A1**
(43) Date of publication of application: **10.04.2024**
(21) Application number: 22811661.2
(22) Date of filing: 26.05.2022
(51) Int. Cl.: C07K 16/40, A61P 25/28, A23L 33/18, A61K 39/00

(54) **PHARMACEUTICAL COMPOSITION FOR TREATING BRAIN DISEASES COMPRISING ANTIBODY SPECIFICALLY BINDING TO ASM PROTEIN AS ACTIVE INGREDIENT**

(30) Priority: 27.05.2021 KR 20210068628; 10.05.2022 KR 20220057049
(71) Applicant: Isu Abxis Co., Ltd., Seongnam-si, Gyeonggi-do 13488 (KR); Kyungpook National University Industry-Academic Cooperation Foundation, Daegu 41566 (KR)
(72) Inventor: HONG, Seung Beom, Gwangju-si Gyeonggi-do 12784 (KR); BAE, Jae Sung, Daegu 42123 (KR); JIN, Hee Kyung, Daegu 42513 (KR)
(74) Representative: Maiwald GmbH
(86) International application number: PCT/KR2022/007485
(87) International publication number: WO 2022/250470

(57) **Abstract**

Disclosed is use of an antibody or antigen-binding fragment thereof that specifically binds to an acid sphingomyelinase (ASM) protein, wherein the antibody or antigen-binding fragment thereof specifically binds to an ASM protein with high binding affinity, significantly inhibits the activity of an ASM protein present in the cellular membrane, and shows effects of improving cognitive memory, inhibiting ASM protein activity, inhibiting the accumulation of amyloid-β and tau proteins, and inhibiting the production of neuroinflammation even without toxicity in Alzheimer's disease animal models, and thus can be advantageously used in the treatment of a brain disease.

## Description

### BACKGROUND OF THE INVENTION

### 1. Field of the invention

The present disclosure relates to use of antibodies specifically binding to acid sphingomyelinase (ASM) protein.

### 2. Description of the Prior Art

Sphingolipid metabolism regulates normal cellular signaling, and abnormal changes in sphingolipid metabolism cause various neurodegenerative diseases including Alzheimer's disease. Acid sphingomyelinase (ASM) protein, an enzyme that regulates sphingolipid metabolism, is a protein expressed in almost all types of cells and plays an important role in sphingolipid metabolism and cell membrane turnover.

In the brain of patients with neurodegenerative diseases such as Alzheimer's disease, the activity of ASM protein is significantly increased compared with that of normal people. In this connection, Korean Patent No. 10-1521117 discloses that the inhibition of the activity of over-expressed ASM protein or the inhibition of the expression into ASM protein suppresses the accumulation of amyloid-β and improves learning ability and memory and thus can treat neurodegenerative diseases. It has been recently known that the activity of ASM protein is also increased in neurological diseases, such as depression, and thus the inhibition of the expression or activity of ASM protein is effective in the amelioration of depression.

However, substances that directly inhibit the expression or activity of ASM protein have not been developed, and several types of inhibitors that indirectly inhibit the expression of ASM proteins have been identified. For example, there are tricyclic antidepressants used in the treatment of depression, and examples thereof include amitriptyline, desipramine, mipramine and the like. Although these tricyclic antidepressants were not developed as ASM protein inhibitors, various studies have demonstrated that such antidepressants exhibit ASM protein inhibitory effects. A major pharmacological mechanism of tricyclic antidepressants is that the activity thereof is increased by inhibiting the reuptake of neurotransmitters in nerve cells, and their action as an ASM inhibitor was confirmed to be a subordinate action. However, tricyclic antidepressants may act on the nervous system and nerve cells, causing side effects, such as blurred vision, increased light sensitivity, and vomiting.

### SUMMARY OF THE INVENTION

An aspect of the present disclosure is to provide use of an antibody specifically binding to ASM protein.

In accordance with aspects of the present disclosure, the present disclosure provides a pharmaceutical composition for preventing or treating a brain disease, the pharmaceutical composition including as an active ingredient an antibody or antigen-binding fragment thereof that specifically binds to an acid sphingomyelinase (ASM) protein.

In accordance with aspects of the present disclosure, the present disclosure provides a health functional food for preventing or alleviating a brain disease, the health functional food including as an active ingredient an antibody or antigen-binding fragment thereof that specifically binds to an ASM protein.

In accordance with aspects of the present disclosure, the present disclosure provides a method for preventing, alleviating, or treating a brain disease, the method including a step of administering to a subject an antibody or antigen-binding fragment thereof that specifically binds to an ASM protein.

In accordance with aspects of the present disclosure, the present disclosure provides an antibody or antigen-binding fragment thereof that specifically binds to an ASM protein for use in a method for preventing, alleviating, or treating a brain disease.

The antibody or antigen-binding fragment thereof according to the present disclosure specifically binds to an ASM protein with high binding affinity, significantly inhibits the activity of an ASM protein present in the cellular membrane, and shows effects of improving cognitive memory, inhibiting ASM protein activity, inhibiting the accumulation of amyloid-β and tau proteins, and inhibiting the production of neuroinflammation even without toxicity in Alzheimer's disease animal models, and thus can be advantageously used in the treatment of a brain disease.

### BRIEF DESCRIPTION OF THE DRAWINGS

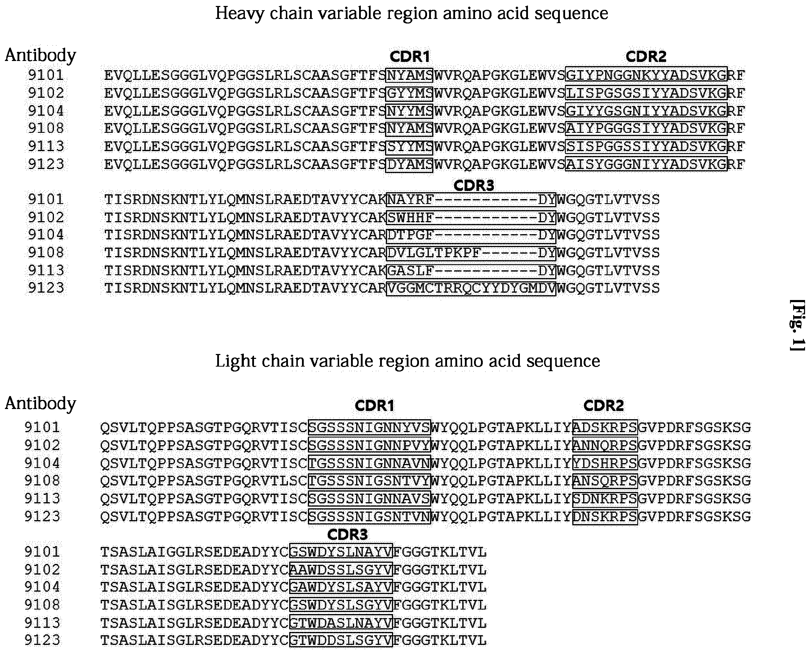

FIG. 1 illustrates a schematic diagram showing amino acid sequences of heavy and light chain variable regions of antibodies produced according to an example of the present disclosure.
FIG. 2 illustrates a graph showing the binding of the antibodies produced according to an example of the present disclosure to ASM protein, as confirmed by ELISA analysis.
FIG. 3 illustrates a schematic diagram showing the antigen binding sites of the antibodies produced according to an example of the present disclosure on ASM protein, in an ASM protein structure model.
FIG. 4 illustrates a deuterium exchange heat map, in the ASM protein saposin domain, of each of the antibodies produced according to an example of the present disclosure.
FIG. 5 illustrates graphs showing the inhibition of activity of an ASM protein located in the cellular membrane of Alzheimer's patients by the antibody produced according to an example of the present disclosure.
FIG. 6 illustrates a graph showing that the antibody produced according to an example of the present disclosure inhibited the activity of an ASM protein located in the cellular membrane of Alzheimer's patients in a concentration-dependent manner.
FIG. 7 illustrates graphs showing the alteration of sphingomyelin metabolism in the cellular membrane of Alzheimer's patients by the antibody produced according to an example of the present disclosure.
FIG. 8 illustrates a schematic diagram showing that the antibody produced according to an example of the present disclosure was administered to Alzheimer's disease animal models.
FIG. 9 illustrates a graph showing the Morris water maze test results in Alzheimer's disease animal models administered with the antibody produced according to an example of the present disclosure.
FIG. 10 illustrates a swimming route of an Alzheimer's disease animal model administered with the antibody produced according to an example of the present disclosure.
FIG. 11 illustrates graphs showing the time spent in target (A0) or non-target (A1, A2, and A3) quadrants of Alzheimer's disease animal models administered with the antibody produced according to an example of the present disclosure.
FIG. 12 illustrates graphs showing the path length (A), swim speed (B), and frequency of crossing platform (C) in Alzheimer's disease animal models administered with the antibody produced according to an example of the present disclosure.
FIG. 13 illustrates graphs showing the contextual and cued fear conditioning test results according to the contextual (A) or tone (B) condition in Alzheimer's disease animal models administered with the antibody produced according to an example of the present disclosure.
FIG. 14 illustrates a graph showing the inhibition of ASM activity in the blood plasma of Alzheimer's disease animal models administered with the antibody produced according to an example of the present disclosure.
FIG. 15 illustrates a graph showing the level of ASM protein in the blood plasma of Alzheimer's disease animal models administered with the antibody produced according to an example of the present disclosure.
FIG. 16 illustrates the expression of dense-core amyloid-β plaques in the brain cortex or hippocampus of Alzheimer's disease animal models administered with the antibody produced according to an example of the present disclosure.
FIG. 17 illustrates the expression of dense-core and diffuse amyloid-β plaques in the brain cortex or hippocampus of Alzheimer's disease animal models administered with the antibody produced according to an example of the present disclosure.
FIG. 18 illustrates graphs showing the expression of amyloid-β40 or amyloid-β42 in the brain cortex or hippocampus of Alzheimer's disease animal models administered with the antibody produced according to an example of the present disclosure.
FIG. 19 illustrates the expression of tau protein in the brain cortex or hippocampus of Alzheimer's disease animal models administered with the antibody produced according to an example of the present disclosure.
FIG. 20 illustrates the expression of lba-1 protein in the brain cortex or hippocampus of Alzheimer's disease animal models administered with the antibody produced according to an example of the present disclosure.
FIG. 21 illustrates the expression of GFAP protein in the brain cortex or hippocampus of Alzheimer's disease animal models administered with the antibody produced according to an example of the present disclosure.
FIG. 22 illustrates graphs showing the changes in survival rate (A) and body weight (B) of Alzheimer's disease animal models administered with the antibody produced according to an example of the present disclosure.
FIG. 23 illustrates organ toxicity in Alzheimer's disease animal models administered with the antibody produced according to an example of the present disclosure.

### BEST MODE FOR INVENTION

Hereinafter, the present disclosure will be described in detail.

The present disclosure provides a pharmaceutical composition for preventing or treating a brain disease, the pharmaceutical composition including as an active ingredient an antibody or antigen-binding fragment thereof that specifically binds to an acid sphingomyelinase (ASM) protein.

As used herein, the term "acid sphingomyelinase (ASM) protein" refers to an enzyme, which is a member of the sphingomyelinase (SMase) family that regulates sphingolipid metabolism. The ASM protein catalyzes the degradation of sphingomyelin into ceramide and phosphorylcholine, and may be classified as an alkaline, neutral, or acidic ASM protein depending on pH at which optimal enzymatic activity thereof is exhibited.

The ASM protein may encompass any type of ASM protein that is known in the art. Specifically, the ASM protein may be derived from a mammal, more specifically, a human, a monkey, a rat, or a mouse. In addition, the ASM protein may contain all the amino acid sequences known as the ASM protein in the art. For example, the ASM protein may be a polypeptide consisting of the amino acid sequences as set forth in SEQ ID NO: 66 and 67, respectively, or a nucleic acid encoding the same.

The ASM protein may be one that has addition, deletion, or substitution of at least one amino acid in the amino acid sequence as set forth in SEQ ID NO: 66 or 67 as long as one retains biological activity equivalent or corresponding to that of the ASM protein. The substitution of the amino acid may be conservative substitution that is performed within a range in which the substitution does not affect or gives a weak effect on the entire protein charge, that is, polarity or hydrophobicity. The ASM protein may also have at least 80%, at least 90%, at least 95%, at least 97%, or at least 99% homology with the amino acid sequences as set forth in SEQ ID NO: 66 and 67, respectively. The ASM protein may contain only a portion thereof as long as the portion retains biological activity equivalent or corresponding to that of the ASM protein.

As used herein, the term "antibody" refers to an immune protein that binds to an antigen to interfere with an action of the antigen or eliminate the antigen. The antibody may include all types of antibodies known in the art. Specifically, the antibody may include IgM, IgD, IgG, IgA, and IgE, which may contain heavy chains formed from µ, δ, γ, α, and ε genes encoding heavy chain constant regions, respectively. In antibody technology, IgG is typically used. IgG may include an isotype, IgG1, IgG2, IgG3, or IgG4, which may be different in terms of structural and functional characteristics. The antibody may include all of a humanized antibody containing a minimal sequence derived from a non-human antibody, a human antibody containing a sequence derived from a human, or a chimeric antibody containing mixed sequences derived from different species.

The IgG may have a very stable Y-shaped structure (about 150 kDa) made of two heavy chain proteins of about 50 kDa and two light chain proteins of about 25 kDa. Heavy and light chains constituting antibodies may be divided into variable regions having amino acid sequences, which are different among antibodies, and constant regions having amino acid sequences, which are same among antibodies. The heavy chain constant regions include CH1, hinge (H), CH2, and CH3 domains, each of which is composed of two β-sheets and which may be linked via an intermolecular disulfide bond. In addition, the light chain constant regions may include a CL domain. Two heavy chain and light chain variable regions are combined to form an antigen-binding site, and one antigen binding site may be present in each of the two Y-shaped arms. In the full-length antibody, a region that can bind to an antigen is called an antibody binding fragment (Fab), and a region that cannot bind to an antigen is called a crystallizable fragment (Fc), and the Fab and Fc may be connected by a hinge. In an embodiment of the present disclosure, the IgG may be a mouse-derived IgG and, specifically, may include a mouse IgG heavy chain constant region consisting of the amino acid sequence as set forth in SEQ ID NO: 74 and a mouse light chain λ constant region consisting of the amino acid sequence as set forth in SEQ ID NO: 76. Alternatively, the mouse-derived IgG may include a mouse IgG heavy chain constant region consisting of the nucleotide sequence as set forth in SEQ ID NO: 75 and a mouse light chain λ constant region containing the nucleotide sequence as set forth in SEQ ID NO: 77.

The antibody according to the present disclosure may include not only the full-length antibody but also an antigen-binding fragment thereof. Specifically, the antigen-binding fragment may refer to a region except for Fc that functions to transmit the stimulus with an antigen to a cell, a complement, or the like. For example, the antigen-binding fragment of the antibody may include all of Fab, scFv, F(ab)2, and Fv, and may also include a 3rd generation antibody fragment, such as a single domain antibody and a minibody.

In an aspect of the present disclosure, the antibody or antigen-binding fragment may bind to at least one epitope that is selected from the group consisting of a fragment of amino acids at positions 53 to 72, a fragment of amino acids at positions 101 to 123, a fragment of amino acids at positions 135 to 159, a fragment of amino acids at positions 135 to 155, a fragment of amino acid at positions 218 to 228, and a fragment of amino acids at positions 259 to 269 from the N-terminus of the ASM protein. The ASM protein may have the features as described above. In an embodiment of the present disclosure, the fragment of amino acids at positions 53 to 72 from the N-terminus of the ASM protein may be a polypeptide consisting of the amino acid sequence as set forth in SEQ ID NO: 68 (TAINLGLKKEPNVARVGSVA); the fragment of amino acids at positions 101 to 123 from the N-terminus of the ASM protein may be a polypeptide consisting of the amino acid sequence as set forth in SEQ ID NO: 69 (VWRRSVLSPSEACGLLLGSTCGH); the fragment of amino acids at positions 135 to 159 from the N-terminus of the ASM protein may be a polypeptide consisting of the amino acid sequence as set forth in SEQ ID NO: 70 (PTVPKPPPKPPSPPAPGAPVSRILF); the fragment of amino acids at positions 135 to 155 from the N-terminus of the ASM protein may be a polypeptide consisting of the amino acid sequence as set forth in SEQ ID NO: 71 (PTVPKPPPKPPSPPAPGAPVS); the fragment of amino acids at positions 218 to 228 from the N-terminus of the ASM protein may be a polypeptide consisting of the amino acid sequence as set forth in SEQ ID NO: 72 (SGLGPAGPFDM); and the fragment of amino acids at positions 259 to 269 from the N-terminus of the ASM protein may be a polypeptide consisting of the amino acid sequence as set forth in SEQ ID NO: 73 (VRKFLGPVPVY). That is, according to another aspect of the present disclosure, the antibody or antigen-binding fragment may bind to at least one epitope selected from the group consisting of the polypeptides sets forth in SEQ ID NOS: 68 to 73, respectively.

As used herein, the term "epitope" refers to a specific site of an antigen that can be recognized by an antibody, and means an antigenic determinant to which an antibody can specifically bind. The epitope is commonly used as the same meaning as a determinant or an antigenic determinant. The epitope consists of chemically active surface groupings of molecules, such as amino acids or sugar side chains, and has usually specific three-dimensional structural as well as specific charge characteristics.

In an aspect of the present disclosure, the antibody or antigen-binding fragment thereof may include: a heavy chain variable region comprising a heavy chain CDR1 (X₁YX₂MS) consisting of the amino acid sequence as set forth in SEQ ID NO: 61, a heavy chain CDR2 (X₃IX₄X₅X₆X₇X₈X₉X₁₀YYADSVKG) consisting of the amino acid sequence as set forth in SEQ ID NO: 62, and a heavy chain CDR3 selected from the group containing of heavy chain CDR3 consisting of amino acid sequences as set forth in SEQ ID NOS: 27, 30, 33, 36, 39, and 42; and a light chain variable region including a light chain CDR1 (X₁₁GSSSNIGX₁₂NX₁₃VX₁₄) consisting of the amino acid sequence as set forth in SEQ ID NO: 63, a light chain CDR2 (X₁₅X₁₆X₁₇X₁₈RPS) consisting of the amino acid sequence as set forth in SEQ ID NO: 64, and a light chain CDR3 (X₁₉X₂₀WDX₂₁SLX₂₂X₂₃YV) consisting of the amino acid sequence as set forth in SEQ ID NO 65.

The term "complementarity determining region (CDR)" refers to a hypervariable region that is in the heavy chain and light chain variable regions of an antibody and has a different amino acid sequence for each antibody, and means a region that actually binds to an antigen. In a three-dimensional conformation of an antibody, CDR has a loop shape on a surface of the antibody, wherein under the loop, a framework region (FR) may exist for structurally supporting CDR. There are three loop structures in each of the heavy chain and the light chain, and these six loop structures can be combined together to directly contact an antigen. The antigen-binding site having six loop structures, CDRs, may be, for convenience, heavy chain CDR1, heavy chain CDR2, heavy chain CDR3, light chain CDR1, light chain CDR2 or light chain CDR3.

For example, in the heavy chain CDR1 (X₁YX₂MS) consisting of the amino acid sequence as set forth in SEQ ID NO: 61, X₁ and X₂ each may be selected from acidic and neutral amino acids. Specifically, X₁ may be an acidic or neutral amino acid and X₂ may be a neutral amino acid. For example, X₁ and X₂ each may be selected from the group consisting of asparagine (Asn), glycine (Gly), serine (Ser), aspartic acid (Asp), alanine (Ala), and tyrosine (Tyr). Specifically, X₁ may be asparagine, glycine, serine, or aspartic acid, and X₂ may be alanine or tyrosine. In an embodiment of the present disclosure, the heavy chain CDR1 may consist of an amino acid sequence as set forth in SEQ ID NO: 25, 28, 31, 34, 37, or 40.

In the heavy chain CDR2 (X₃IX₄X₅X₆X₇X₈X₉X_{I0}YYADSVKG) consisting of the amino acid sequence of SEQ ID NO: 62, X₃ to X₁₀ each may be selected from neutral and basic amino acids. Specifically, X₃ and X₄ to X₉ each may be a neutral amino acid, and X₁₀ may be a neutral or basic amino acid. For example, X₃ to X₁₀ each may be selected from the group consisting of glycine, leucine (Leu), alanine, serine, tyrosine, proline (Pro), asparagine, lysine (Lys), and isoleucine (Ile). Specifically, X₃ may be glycine, leucine, alanine, or serine; X₄ may be tyrosine or serine; X₅ may be proline or tyrosine; X₆ may be asparagine or glycine; X₇ and X₈ each may be glycine or serine; X₉ may be asparagine or serine; and X₁₀ may be lysine or isoleucine. In an embodiment of the present disclosure, the heavy chain CDR2 may consist of an amino acid sequence as set forth in SEQ ID NO: 26, 29, 32, 35, 38, or 41.

In the light chain CDR1 (X₁₁GSSSNIGX₁₂NX₁₃VX₁₄) consisting of the amino acid sequence as set forth in SEQ ID NO: 63, X₁₁ to X₁₄ each may be selected from neutral amino acids. For example, X₁₁ to X₁₄ each may be selected from the group consisting of threonine (Thr), serine, asparagine, alanine, proline, and tyrosine. Specifically, X₁₁ may be threonine or serine; X₁₂ may be asparagine or serine; X₁₃ may be alanine, proline, threonine, or tyrosine; and X₁₄ may be asparagine, tyrosine, or serine. In an embodiment of the present disclosure, the light chain CDR1 may consist of an amino acid sequence as set forth in SEQ ID NO: 43, 46, 49, 52, 55, or 58.

In the light chain CDR2 (X₁₅X₁₆X₁₇X₁₈RPS) consisting of the amino acid sequence as set forth in SEQ ID NO: 64, X₁₅ to X₁₈ each may be selected from acidic, neutral, and basic amino acids. Specifically, X₁₅ and X₁₆ each may be an acidic or neutral amino acid, and X₁₇ may be a neutral amino acid, and X₁₈ may be a basic or neutral amino acid. For example, X₁₅ to X₂₈ each may be selected from the group consisting of tyrosine, alanine, aspartic acid, serine, asparagine, histidine (His), glutamine (Gln), and lysine. Specifically, X₁₅ may be tyrosine, alanine, aspartic acid, or serine; X₁₆ may be aspartic acid or asparagine; X₁₇ may be serine or asparagine; and X₁₈ may be histidine, glutamine, or lysine. In an embodiment of the present disclosure, the light chain CDR2 may consist of an amino acid sequence as set forth in SEQ ID NO: 44, 47, 50, 53, 56, or 59.

In the light chain CDR3 (X₁₉X₂₀WDX₂₁SLX₂₂X₂₃YV) consisting of the amino acid sequence as set forth in SEQ ID NO: 65, X₁₉ to X₂₃ each may be selected from neutral amino acids. For example, X₁₉ to X₂₃ each may be selected from the group consisting of glycine, alanine, serine, threonine, tyrosine, aspartic acid, and asparagine. Specifically, X₁₉ may be glycine or alanine; X₂₀ may be alanine, serine, or threonine; X₂₁ may be tyrosine, serine, alanine, or aspartic acid; X₂₂ may be serine or asparagine; and X₂₃ may be alanine or glycine. In an embodiment of the present disclosure, the light chain CDR3 may consist of an amino acid sequence as set forth in SEQ ID NO: 45, 48, 51, 54, 57, or 60.

In another aspect of the present disclosure, the antibody or antigen-binding fragment thereof may include: a heavy chain variable region including, a heavy chain CDR1 selected from the group consisting of an amino acid sequence as set forth in SEQ ID NOS: 25, 28, 31, 34, 37, and 40, respectively, a heavy chain CDR2 selected from the group consisting of amino acid sequences as set forth in SEQ ID NOS: 26, 29, 32, 35, 38, and 41, respectively, and a heavy chain CDR3 selected from the group consisting of amino acid sequences as set forth in SEQ ID NOS: 27, 30, 33, 36, 39, and 42, respectively; and a light chain variable region including, a light chain CDR1 selected from the group consisting of amino acid sequences as set forth in SEQ ID NOS: 43, 46, 49, 52, 55, and 58, respectively, a light chain CDR2 selected from the group consisting of amino acid sequences as set forth in SEQ ID NOS: 44, 47, 50, 53, 56, and 59, respectively, and a light chain CDR3 selected from the group consisting of amino acid sequences as set forth in SEQ ID NOS: 45, 48, 51, 54, 57, and 60, respectively.

In addition, the antibody or antigen-binding fragment thereof may include: a heavy chain variable region selected from the group composing of, a heavy chain variable region including heavy chain CDR1, CDR2, and CDR3 consisting of the amino acid sequences as set forth in SEQ ID NOS: 25, 26, and 27, respectively, a heavy chain variable region including heavy chain CDR1, CDR2, and CDR3 consisting of the amino acid sequences as set forth in SEQ ID NOS: 28, 29, and 30, respectively, a heavy chain variable region including heavy chain CDR1, CDR2, and CDR3 consisting of the amino acid sequences as set forth in SEQ ID NOS: 31, 32, and 33, respectively, a heavy chain variable region including heavy chain CDR1, CDR2, and CDR3 consisting of the amino acid sequences as set forth in SEQ ID NOS: 34, 35, and 36, respectively, a heavy chain variable region including heavy chain CDR1, CDR2, and CDR3 consisting of the amino acid sequences as set forth in SEQ ID NOS: 37, 38, and 39, respectively, and a heavy chain variable region including heavy chain CDR1, CDR2, and CDR3 consisting of the amino acid sequences as set forth in SEQ ID NOS: 40, 41, and 42, respectively; and a light chain variable region selected from the group composing of, a light chain variable region including light chain CDR1, CDR2, and CDR3 consisting of the amino acid sequences as set forth in SEQ ID NOS: 43, 44, and 45, respectively, a light chain variable region including light chain CDR1, CDR2, and CDR3 consisting of the amino acid sequences as set forth in SEQ ID NOS: 46, 47, and 48, respectively, a light chain variable region including light chain CDR1, CDR2, and CDR3 consisting of the amino acid sequences as set forth in SEQ ID NOS: 49, 50, and 51, respectively, a light chain variable region including light chain CDR1, CDR2, and CDR3 consisting of the amino acid sequences as set forth in SEQ ID NOS: 52, 53, and 54, respectively, a light chain variable region including light chain CDR1, CDR2, and CDR3 consisting of the amino acid sequences as set forth in SEQ ID NOS: 55, 56, and 57, respectively, and a light chain variable region including light chain CDR1, CDR2, and CDR3 consisting of the amino acid sequences as set forth in SEQ ID NOS: 58, 59, and 60, respectively.

Furthermore, the antibody or antigen-binding fragment thereof may include: a heavy chain variable region including heavy chain CDR1, CDR2, and CDR3 consisting of the amino acid sequences as set forth in SEQ ID NOS: 25, 26, and 27, respectively, and a light chain variable region including light chain CDR1, CDR2, and CDR3 consisting of the amino acid sequences as set forth in SEQ ID NOS: 43, 44, and 45, respectively; a heavy chain variable region including heavy chain CDR1, CDR2, and CDR3 consisting of the amino acid sequences as set forth in SEQ ID NOS: 28, 29, and 30, respectively, and a light chain variable region including light chain CDR1, CDR2, and CDR3 consisting of the amino acid sequences as set forth in SEQ ID NOS: 46, 47, and 48, respectively; a heavy chain variable region including heavy chain CDR1, CDR2, and CDR3 consisting of the amino acid sequences as set forth in SEQ ID NOS: 31, 32, and 33, respectively, and a light chain variable region including light chain CDR1, CDR2, and CDR3 consisting of the amino acid sequences as set forth in SEQ ID NOS: 49, 50, and 51, respectively; a heavy chain variable region including heavy chain CDR1, CDR2, and CDR3 consisting of the amino acid sequences as set forth in SEQ ID NOS: 34, 35, and 36, respectively, and a light chain variable region including light chain CDR1, CDR2, and CDR3 consisting of the amino acid sequences as set forth in SEQ ID NOS: 52, 53, and 54, respectively; a heavy chain variable region including heavy chain CDR1, CDR2, and CDR3 consisting of the amino acid sequences as set forth in SEQ ID NOS: 37, 38, and 39, respectively, and a light chain variable region including light chain CDR1, CDR2, and CDR3 consisting of the amino acid sequences as set forth in SEQ ID NOS: 55, 56, and 57, respectively; or a heavy chain variable region including heavy chain CDR1, CDR2, and CDR3 consisting of the amino acid sequences as set forth in SEQ ID NOS: 40, 41, and 42, respectively, and a light chain variable region including light chain CDR1, CDR2, and CDR3 consisting of the amino acid sequences as set forth in SEQ ID NOS: 58, 59, and 60, respectively.

For example, the heavy chain variable region may be a polypeptide consisting of an amino acid sequence as set forth in SEQ ID NO: 1, 3, 5, 7, 9, or 11, and the light chain variable region may be a polypeptide consisting of an amino acid sequence as set forth in SEQ ID NO: 13, 15, 17, 19, 21, or 23.

In addition, the antibody or antigen-binding fragment thereof according to the present disclosure may be modified as needed. Specifically, the antibody or antigen-binding fragment thereof may be modified by conjugation, glycosylation, tagging, or a combination thereof. Specifically, the antibody or antigen-binding fragment thereof may be modified with horseradish peroxidase (HRP), an alkaline phosphatase, hapten, biotin, streptavidin, a fluorescent substance, a radioactive substance, a quantum dot, polyethylene glycol (PEG), a histidine tag, or the like. The antibody or antigen-binding fragment thereof may also be conjugated to another drug as needed.

The antibody or antigen-binding fragment thereof may be produced according to a monoclonal antibody production method known in the art, and the production method may be appropriately modified by a person skilled in the art. For example, the antibody may be produced by preparing hybridomas through the use of B lymphocytes obtained from an animal immunized with an antigen, or may be produced by phage display technology.

In another aspect of the present disclosure, the antibody or antigen-binding fragment thereof included in the pharmaceutical composition according to the present disclosure may include a nucleic acid encoding the antibody or antigen-binding fragment thereof. Since the amino acid sequence constituting the antibody or antigen-binding fragment thereof is known, a nucleic acid sequence encoding the same would also be obvious to a person skilled in the art. The nucleic acid sequence may have at least one nucleotide addition, deletion, or substitution as long as the activity of the antibody or antigen-binding fragment translated thereof is retained.

In still another aspect of the present disclosure, the pharmaceutical composition according to the present disclosure may include an expression vector including a nucleic acid encoding the antibody or antigen-binding fragment thereof. The term "expression vector" is a means for expressing a target gene in a host cell, and may include all of a plasmid vector, a cosmid vector, a bacteriophage vector, a viral vector, and the like. The expression vector may include elements necessary for preparing the peptide from the nucleic acid included therein. Specifically, the expression vector may include a signal sequence, an origin of replication, a marker gene, a promoter, a transcription termination sequence, and the like. The nucleic acid encoding the antibody or antigen-binding fragment thereof according to the present disclosure may be operatively linked to the promoter.

As an example, an expression vector used in prokaryotic cells may include a promoter for transcription, a ribosome binding site for initiation of translation, and termination sequences for transcription and translation. The expression vector used in eukaryotic cells may include a promoter and polyadenylation sequence derived from a mammal or a mammalian virus.

All marker genes known in the art may be used as a marker gene included in the expression vector, and may be specifically an antibiotic resistant gene. Specifically, the antibiotic resistant gene may be a gene showing resistance to antibiotics including ampicillin, gentamicin, carbenicillin, chloramphenicol, streptomycin, kanamycin, neomycin, tetracycline, and the like.

In still another aspect of the present disclosure, the pharmaceutical composition according to the present disclosure may include as an active ingredient a host cell including the nucleic acid or expression vector as described above. All types of cells known to be usable to produce antibodies or antigen-binding fragments thereof in the art may be used as a host cell. Specifically, the host cell may be a prokaryotic cell, yeast, or a eukaryotic cell. Examples of the prokaryotic cell may include *E. coli,* the genus Bacillus strains, the genus Streptomyces strain, the genus Pseudomonas strain, the genus Staphylococcus strain, and the like, and examples of the yeast may include Saccharomyces cerevisiae and the like. Example of the eukaryotic cell may include COS-7, BHK, CHO, CHOK1, DXB-11, DG-44, CHO/-DHFR, CV1, HEK293, TM4, VERO, HELA, MDCK, BRL 3A, W138, Hep G2, SK-Hep, MMT, TRI, MRC 5, FS4, 3T3, RIN, A549, PC12, K562, PERC6, SP2/0, NS-0, U20S, HT1080, and the like.

The host cell may be transduced with the nucleic acid or expression vector as described above according to a method known in the art. Specifically, the transduction may be performed by transient transfection, microinjection, transduction, cell infusion, calcium phosphate precipitation, liposome-mediated transfection, DEAE dextran-mediated transfection, polybrene-mediated transfection, electroporation, a gene gun, or the like. The methods may also be appropriately modified by a person skilled in the art.

The brain disease may include all types of brain diseases known in the art and, specifically, the brain disease may be a degenerative brain disease. As an example, the degenerative brain disease may be a disease in which the expression or aggregation level of amyloid-β is higher or at risk of being higher than normal. For example, the degenerative brain disease may be dementia, Alzheimer's disease, Parkinson's disease, Huntington's disease, mild cognitive impairment, cerebral amyloid angiopathy, Down syndrome, amyloid stroke, systemic amyloid disease, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, or frontotemporal dementia.

The pharmaceutical composition according to the present disclosure may include an antibody or antigen-binding fragment thereof, which is an active ingredient and specifically binds to an ASM protein, in 10 to 95 wt% relative to the total weight thereof. In addition, the pharmaceutical composition of the present disclosure may further include, in addition to the above active ingredient, at least one active ingredient that exhibits the same or similar function to the above ingredient.

The pharmaceutical composition of the present disclosure may include a carrier, a diluent, an excipient, or a mixture thereof that is commonly used in biological preparations. Any pharmaceutically acceptable carrier may be used as long as it is suitable for *in vivo* delivery of the composition. Specifically, the carrier may be a compound, saline, sterile water, Ringer's solution, a dextrose solution, a maltodextrin solution, glycerol, ethanol, or a mixture thereof, described in Merck Index, 13th ed., Merck Co. Inc. In addition, common additives, such as an antioxidant, a buffer, and a bacteriostatic agent, may be added as needed.

When the composition is formulated into a preparation, a diluent or an excipient that is commonly used, such as a filler, an extender, a binder, a wetting agent, a disintegrant, or a surfactant may be added.

The composition of the present disclosure may be formulated into an oral preparation or a parenteral preparation. Examples of the oral preparation may include a solid preparation and a liquid preparation. The solid preparation may be a tablet, a pill, a powder, granules, a capsule, or a troche, and these solid preparations may be prepared by adding at least one excipient to the composition. The excipient may be starch, calcium carbonate, sucrose, lactose, gelatin, or a mixture thereof. The solid preparation may include a lubricant, and examples thereof may be magnesium stearate, talc, and the like. The liquid formulation may be a suspension, an oral liquid, an emulsion, or a syrup. The liquid formulation may include an excipient, such as a wetting agent, a sweetener, an aromatic agent, or a preservative, and the like.

Examples of the parenteral preparation may include an injection, a suppository, a respiratory inhalation powder, a spray aerosol, a powder, a cream, and the like. The injection may include a sterile aqueous solution, a non-aqueous solvent, a suspension, an emulsion, and the like. Examples of the non-aqueous solvent or suspension may use propylene glycol, polyethylene glycol, a vegetable oil such as olive oil, an injectable ester such as ethylolate, and the like.

Furthermore, the present disclosure provides a health functional food for preventing or alleviating a brain disease, the health functional food including as an active ingredient an antibody or antigen-binding fragment thereof that specifically binds to an ASM protein.

The antibody or antigen-binding fragment thereof that specifically binds to an ASM protein and included as an active ingredient in the health functional food according to the present disclosure may have the characteristics as described above.

The antibody or antigen-binding fragment thereof that specifically binds to an ASM protein of the present disclosure may be added as it is to a food or may be used along with other foods or food ingredients. The content of the added active ingredient may be determined according to the purpose, and may be usually 0.01 to 90 parts by weight relative to the total weight of the food.

The form and kind of health functional food are not particularly limited. Specifically, the health functional food may be in the forms of a tablet, a capsule, a powder, granules, a liquid, and a pill. The health functional food may include as additional ingredients, several flavoring agents, sweeteners, or natural carbohydrates. The sweetener may be a natural or synthetic sweetener, and examples of the natural sweetener include thaumatin, a stevia extract, and the like. Examples of the synthetic sweetener are saccharin, aspartame, and the like. In addition, the natural carbohydrates may be monosaccharides, disaccharides, polysaccharides, oligosaccharides, and sugar alcohols.

The health functional food of the present disclosure may further include, in addition to the above-described additional ingredients, nutritional supplements, vitamins, electrolytes, flavors, colorants, pectic acid and salts thereof, alginic acid and salts thereof, organic acids, protective colloidal thickeners, pH adjusters, stabilizers, preservatives, glycerin, alcohols, and the like. These ingredients may be used either alone or in combination. The proportion of such an additive may be selected within a range of 0.01 to 0.1 parts by weight relative to 100 parts by weight of the composition of the present disclosure.

In accordance with aspects of the present disclosure, the present disclosure provides a method for preventing, alleviating, or treating a brain disease, the method including a step of administering to a subject an antibody or antigen-binding fragment thereof that specifically binds to an ASM protein.

The antibody or antigen-binding fragment thereof that specifically binds to an ASM protein administered for preventing, alleviating, or treating a brain disease according to the present disclosure may have the characteristics as described above.

The subject may be a mammal and particularly a human.

The administration may be conducted orally or parenterally according to the desired method. Examples of the parenteral administration may include an intraperitoneal, rectal, subcutaneous, intravenous, intramuscular, or intrathoracic injection.

In addition, the active ingredient may be administered in a pharmaceutically effective amount. The pharmaceutically effective amount may vary depending on the type or severity of disease, the activity of a drug, the sensitivity of a patient to a drug, the time of administration, the route of administration, the duration of treatment, a drug to be used with the composition, and the like. However, for desirable effects, the amount of the active ingredient included in the pharmaceutical composition according to the present disclosure may be 0.0001 to 1,000 mg/kg, specifically 0.001 to 500 mg/kg. The administration may be performed once or several times a day.

The administration may be alone or in combination with other medicines. In the combinative administration, the administration may be performed sequentially or simultaneously.

In accordance with aspects of the present disclosure, the present disclosure provides an antibody or antigen-binding fragment thereof that specifically binds to an ASM protein for use in a method for preventing, alleviating, or treating a brain disease.

The antibody or antigen-binding fragment thereof that specifically binds to an ASM protein for use in a method for preventing, alleviating, or treating a brain disease according to the present disclosure may have the characteristics as described above.

### MODE FOR CARRYING OUT THE INVENTION

Hereinafter, the present disclosure will be described in detail by the following examples. However, the following exemplary embodiments are merely for illustrating the present disclosure, and are not intended to limit the scope of the present disclosure. Any embodiment that has substantially the same constitution as the technical idea described in the claims of the disclosure and achieves the same effects of action are included in the technical scope of the present invention.

### Example 1: Production of Antibodies Specifically Binding to Acid Sphingomyelinase (ASM) Protein

Antibodies that specifically bind to human ASM protein (SEQ ID NO: 66) and mouse ASM protein (SEQ ID NO: 67) were produced as follows.

Specifically, phages with scFv specifically binding to the human ASM protein or mouse ASM protein were selected by performing panning through a typical method using a recombinant human and mouse ASM protein and the human synthetic scFv-phage library. Nucleic acid sequences encoding the selected scFv were analyzed, and amino acid sequences translated therefrom were identified. Expression vectors were produced such that a mouse heavy chain constant region and a mouse light chain λ constant region consisting of the nucleotide sequences as set forth in SEQ ID NOS: 75 and 77, respectively, were linked to the carboxy termini of the heavy chain variable region and the light chain variable region of each of the identified scFv sequences. The amino acid sequences and nucleic acid sequences of the heavy chain variable regions constituting scFv contained in the produced expression vectors are shown in Table 1, and the amino acid sequences and nucleic acid sequences of the light chain variable regions thereof are disclosed in Table 2.

**TABLE 1**

| Antibody # | Sequence | Sequence Number |
|---|---|---|
| #9101 | EVQLLESGGGLVQPGGSLRLSCAASGFTFSNYAMSWVRQAP GKGLEWVSGIYPNGGNKYYADSVKGRFTISRDNSKNTLYLQ MNSLRAEDTAVYYCAKNAYRFDYWGQGTLVTVSS | SEQ ID NO: 1 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATGCTATGAGCTGGGTCCGCCAGGCTCCA GGGAAGGGGCTGGAGTGGGTCTCAGGGATCTATCCTAATGG TGGTAATAAATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAAAATGCTTATCGTTTCGACTACTGGGGCCAGGGTA CACTGGTCACCGTGAGCTCA | SEQ ID NO: 2 |
| #9102 | EVQLLESGGGLVQPGGSLRLSCAASGFTFSGYYMSWVRQAP GKGLEWVSLISPGSGSIYYADSVKGRFTISRDNSKNTLYLQ MNSLRAEDTAVYYCAKSWHHFDYWGQGTLVTVSS | SEQ ID NO: 3 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCGGTTATTATATGAGCTGGGTCCGCCAGGCTCCA GGGAAGGGGCTGGAGTGGGTCTCATTGATCTCTCCTGGTAG TGGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAATCTTGGCATCATTTCGACTACTGGGGCCAGGGTA CACTGGTCACCGTGAGCTCA | SEQ ID NO: 4 |
| #9104 | EVQLLESGGGLVQPGGSLRLSCAASGFTFSNYYMSWVRQAP GKGLEWVSGIYYGSGNIYYADSVKGRFTISRDNSKNTLYLQ MNSLRAEDTAVYYCARDTPGFDYWGQGTLVTVSS | SEQ ID NO: 5 |
| | GAGGTGCAGCTGTTGGAGTCTGGGGGAGGCTTGGTACAGCC | SEQ ID NO: 6 |
| | TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTAG TGGTAATATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | |
| #9108 | EVQLLESGGGLVQPGGSLRLSCAASGFTFSNYAMSWVRQAP GKGLEWVSAIYPGGGSIYYADSVKGRFTISRDNSKNTLYLQ MNSLRAEDTAVYYCARDVLGLTPKPFDYWGQGTLVTVSS | SEQ ID NO: 7 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATGCTATGAGCTGGGTCCGCCAGGCTCCA GGGAAGGGGCTGGAGTGGGTCTCAGCGATCTATCCTGGTGG TGGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATGTTTTGGGTCTGACTCCTAAGCCGTTCGACT ACTGGGGCCAGGGTACACTGGTCACCGTGAGCTCA | SEQ ID NO: 8 |
| #9113 | EVQLLESGGGLVQPGGSLRLSCAASGFTFSSYYMSWVRQAP GKGLEWVSSISPGGSSIYYADSVKGRFTISRDNSKNTLYLQ MNSLRAEDTAVYYCAKGASLFDYWGQGTLVTVSS | SEQ ID NO: 9 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAGTTATTATATGAGCTGGGTCCGCCAGGCTCCA GGGAAGGGGCTGGAGTGGGTCTCATCGATCTCTCCTGGTGG TAGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAAGGGGCGTCTCTGTTCGACTACTGGGGCCAGGGTA CACTGGTCACCGTGAGCTCA | SEQ ID NO: 10 |
| #9123 | EVQLLESGGGLVQPGGSLRLSCAASGFTFSDYAMSWVRQAP GKGLEWVSAISYGGGNIYYADSVKGRFTISRDNSKNTLYLQ MNSLRAEDTAVYYCARVGGMCTRRQCYYDYGMDVWGQGTLV TVSS | SEQ ID NO: 11 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCGATTATGCTATGAGCTGGGTCCGCCAGGCTCCA GGGAAGGGGCTGGAGTGGGTCTCAGCGATCTCTTATGGTGG TGGTAATATATATTACGCTGATTCTGTAAAAGGTCGGTTCA CCATCTCCAGAGACAATTCCAAGAACACGCTGTATCTGCAA | SEQ ID NO: 12 |
| | ATGAACAGCCTGAGAGCCGAGGACACGGCCGTGTATTACTG TGCGAGAGTTGGTGGTATGTGTACTAGGCGTCAGTGTTATT ATGATTATGGTATGGACGTCTGGGGCCAGGGTACACTGGTC ACCGTGAGCTCA | |

**TABLE 2**

| Antibody # | Sequence | SEQ ID NO |
|---|---|---|
| #9101 | QSVLTQPPSASGTPGQRVTISCSGSSSNIGNNYVSWYQQLPG TAPKLLIYADSKRPSGVPDRFSGSKSGTSASLAIGGLRSEDE ADYYCGSWDYSLNAYVFGGGTKLTVL | SEQ ID NO: 13 |
| | CAGTCTGTGCTGACTCAGCCACCCTCAGCTAGCGGGACCCCC GGGCAGAGGGTCACCATCTCTTGTAGTGGCTCTTCATCCAAT ATTGGCAATAATTATGTCTCCTGGTACCAGCAGCTCCCAGGA ACGGCCCCCAAACTCCTCATCTATGCTGATAGTAAGCGGCCA AGCGGGGTCCCTGACCGATTCTCTGGCTCCAAGTCTGGCACC TCAGCCTCGCTGGCCATCGGTGGGCTCCGGTCCGAGGATGAG GCTGATTATTACTGTGGTTCTTGGGATTATAGCCTGAATGCT TATGTCTTCGGCGGAGGCACCAAGCTTACGGTCCTA | SEQ ID NO: 14 |
| #9102 | QSVLTQPPSASGTPGQRVTISCSGSSSNIGNNPVYWYQQLPG TAPKLLIYANNQRPSGVPDRFSGSKSGTSASLAISGLRSEDE ADYYCAAWDSSLSGYVFGGGTKLTVL | SEQ ID NO: 15 |
| | CAGTCTGTGCTGACTCAGCCACCCTCAGCTAGCGGGACCCCC GGGCAGAGGGTCACCATCTCTTGTAGTGGCTCTTCATCTAAT ATTGGCAATAATCCTGTCTACTGGTACCAGCAGCTCCCAGGA ACGGCCCCCAAACTCCTCATCTATGCTAATAATCAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGCTGCTTGGGATTCTAGCCTGAGTGGT TATGTCTTCGGCGGAGGCACCAAGCTTACGGTCCTA | SEQ ID NO: 16 |
| #9104 | QSVLTQPPSASGTPGQRVTISCTGSSSNIGNNAVNWYQQLPG TAPKLLIYYDSHRPSGVPDRFSGSKSGTSASLAISGLRSEDE ADYYCGAWDYSLSAYVFGGGTKLTVL | SEQ ID NO: 17 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTCATCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 18 |
| #9108 | QSVLTQPPSASGTPGQRVTLSCTGSSSNIGSNTVYWYQQLPG TAPKLLIYANSQRPSGVPDRFSGSKSGTSASLAISGLRSEDE ADYYCGSWDYSLSGYVFGGGTKLTVL | SEQ ID NO: 19 |
| | CAGTCTGTGCTGACTCAGCCACCCTCAGCTAGCGGGACCCCC GGGCAGAGGGTCACCCTCTCTTGTACTGGCTCTTCATCTAAT ATTGGCAGTAATACTGTCTACTGGTACCAGCAGCTCCCAGGA ACGGCCCCCAAACTCCTCATCTATGCTAATAGTCAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTTCTTGGGATTATAGCCTGAGTGGT TATGTCTTCGGCGGAGGCACCAAGCTTACGGTCCTA | SEQ ID NO: 20 |
| #9113 | QSVLTQPPSASGTPGQRVTISCSGSSSNIGNNAVSWYQQLPG TAPKLLIYSDNKRPSGVPDRFSGSKSGTSASLAISGLRSEDE ADYYCGTWDASLNAYVFGGGTKLTVL | SEQ ID NO: 21 |
| | CAGTCTGTGCTGACTCAGCCACCCTCAGCTAGTGGGACCCCC GGGCAGAGGGTCACCATCTCTTGTAGTGGCTCTTCATCTAAT ATTGGCAATAATGCTGTCTCCTGGTACCAGCAGCTCCCAGGA ACGGCCCCCAAACTCCTCATCTATTCTGATAATAAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTACTTGGGATGCTAGCCTGAATGCT TATGTCTTCGGCGGAGGCACCAAGCTTACGGTCCTA | SEQ ID NO: 22 |
| #9123 | QSVLTQPPSASGTPGQRVTISCSGSSSNIGSNTVNWYQQLPG TAPKLLIYDNSKRPSGVPDRFSGSKSGTSASLAISGLRSEDE ADYYCGTWDDSLSGYVFGGGTKLTVL | SEQ ID NO: 23 |
| | CAGTCTGTGCTGACTCAGCCACCCTCAGCTAGCGGGACCCCC GGGCAGAGGGTCACCATCTCTTGTAGTGGCTCTTCATCTAAT ATTGGCAGTAATACTGTCAACTGGTACCAGCAGCTCCCAGGA ACGGCCCCCAAACTCCTCATCTATGATAATAGTAAGCGGCCA AGCGGGGTCCCTGACCGATTCTCTGGCTCCAAGTCTGGCACC TCAGCCTCCCTGGCCATCAGTGGGCTCCGGTCCGAAGATGAG GCTGATTATTACTGTGGTACTTGGGATGATAGCCTGAGTGGT TATGTCTTCGGCGGAGGCACCAAGCTTACGGTCCTA | SEQ ID NO: 24 |

The expression vector employed a mammalian expression vector. The produced expression vectors were transformed into the CHO or HEK293 cell line to produce full-length antibodies binding to human ASM protein, or to both human and mouse ASM proteins.

### Example 2: Determination of Complementarity Determining Regions (CDRs)

The complementarity determining regions in the produced scFv fragments were identified by a conventional method, and as a result, the CDR sequences of the heavy chain variable regions are shown in Table 3 and the CDR sequences of the light chain variable regions are shown in Table 4.

**TABLE 3**

| Antibody # | CDR | Sequence | SEQ ID NO |
|---|---|---|---|
| #9101 | CDR1 | NYAMS | SEQ ID NO: 25 |
| | CDR2 | GIYPNGGNKYYADSVKG | SEQ ID NO: 26 |
| | CDR3 | NAYRFDY | SEQ ID NO: 27 |
| #9102 | CDR1 | GYYMS | SEQ ID NO: 28 |
| | CDR2 | LISPGSGSIYYADSVKG | SEQ ID NO: 29 |
| | CDR3 | SWHHFDY | SEQ ID NO: 30 |
| #9104 | CDR1 | NYYMS | SEQ ID NO: 31 |
| | CDR2 | GIYYGSGNIYYADSVKG | SEQ ID NO: 32 |
| | CDR3 | DTPGFDY | SEQ ID NO: 33 |
| #9108 | CDR1 | NYAMS | SEQ ID NO: 34 |
| | CDR2 | AIYPGGGSIYYADSVKG | SEQ ID NO: 35 |
| | CDR3 | DVLGLTPKPFDY | SEQ ID NO: 36 |
| #9113 | CDR1 | SYYMS | SEQ ID NO: 37 |
| | CDR2 | SISPGGSSIYYADSVKG | SEQ ID NO: 38 |
| | CDR3 | GASLFDY | SEQ ID NO: 39 |
| #9123 | CDR1 | DYAMS | SEQ ID NO: 40 |
| | CDR2 | AISYGGGNIYYADSVKG | SEQ ID NO: 41 |
| | CDR3 | VGGMCTRRQCYYDYGMDV | SEQ ID NO: 42 |

**TABLE 4**

| Antibody # | CDR | Sequence | SEQ ID NO |
|---|---|---|---|
| #9101 | CDR1 | SGSSSNIGNNYVS | SEQ ID NO: 43 |
| | CDR2 | ADSKRPS | SEQ ID NO: 44 |
| | CDR3 | GSWDYSLNAYV | SEQ ID NO: 45 |
| #9102 | CDR1 | SGSSSNIGNNPVY | SEQ ID NO: 46 |
| | CDR2 | ANNQRPS | SEQ ID NO: 47 |
| | CDR3 | AAWDSSLSGYV | SEQ ID NO: 48 |
| #9104 | CDR1 | TGSSSNIGNNAVN | SEQ ID NO: 49 |
| | CDR2 | YDSHRPS | SEQ ID NO: 50 |
| | CDR3 | GAWDYSLSAYV | SEQ ID NO: 51 |
| #9108 | CDR1 | TGSSSNIGSNTVY | SEQ ID NO: 52 |
| | CDR2 | ANSQRPS | SEQ ID NO: 53 |
| | CDR3 | GSWDYSLSGYV | SEQ ID NO: 54 |
| #9113 | CDR1 | SGSSSNIGNNAVS | SEQ ID NO: 55 |
| | CDR2 | SDNKRPS | SEQ ID NO: 56 |
| | CDR3 | GTWDASLNAYV | SEQ ID NO: 57 |
| #9123 | CDR1 | SGSSSNIGSNTVN | SEQ ID NO: 58 |
| | CDR2 | DNSKRPS | SEQ ID NO: 59 |
| | CDR3 | GTWDDSLSGYV | SEQ ID NO: 60 |

### Experimental Example 1: Confirmation of Binding to ASM Protein

The binding of the produced antibodies, specifically binding to the ASM protein, to the ASM protein were analyzed by ELISA.

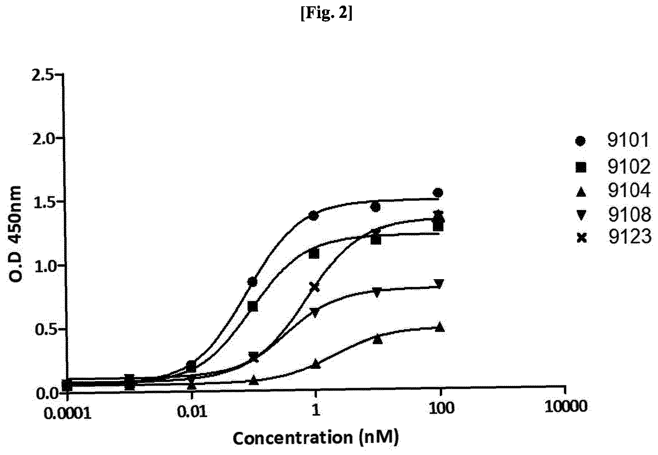

First, 100 µl of 1 µg/mL recombinant human ASM protein (R&D systems) was added to a multi-array 96-well plate (Thermo scientific) and left at 4°C for 16 hours to coat the plate. Then, 200 µl of PBS containing 5% BSA was added to the coated plate, followed by incubation at 37°C for about 2 hours, and then the plate was washed three times with PBS containing 0.05% Tween 20. The plate was treated with the anti-ASM antibody at 0.0001, 0.001, 0.01, 0.1, 1, 10, or 100 nM, followed by incubation at 37°C for about 1 hours. After the incubation, the plate was washed three times with PBS containing 0.05% Tween, and 100 µl of goat-anti-mouse IgG-HRP antibody (Jackson Immunoresearch) as a secondary antibody was added. After incubation again at 37°C for about 1 hour, the plate was washed three times with PBS containing 0.05% Tween. Then, 100 µl of a tetramethylbenzidine (TMB) substrate was added thereto, followed by incubation at room temperature for additional about 5 minutes, and then the reaction was terminated by 100 µl of a 2 N sulfuric acid solution, and the absorbance was measured at a wavelength of 450 nm. As a result, the measured absorbance was shown in FIG. 2 and the EC₅₀ values of antibodies calculated from the absorbance values are shown in Table 5.

**TABLE 5**

| Antibody # | EC₅₀ (nM) |
|---|---|
| #9101 | 0.0815 |
| #9102 | 0.0954 |
| #9104 | 1.948 |
| #9108 | 0.3269 |
| #9123 | 0.7578 |

As shown in FIG. 2, all of the five types of antibodies produced above were bound to the ASM protein in a concentration-dependent manner. In particular, as shown in Table 5, antibody #9101 showed the lowest EC₅₀ value and thus had the highest binding ability to ASM protein.

### Experimental Example 2: Confirmation of Binding Affinity with ASM Protein

The binding affinity of the produced antibodies, specifically binding to the ASM protein, with the ASM protein and the interactive dynamics therebetween were measured using an Octet^{®} QK384 system (Pall Life Sciences).

First, 20 mM 1-ethyl-3-(3-dimethylaminopropyl)carbodiimide hydrochloride (EDC) and 40 mM N-hydroxysulfosuccinimide (sulfo-NHS) solutions were treated on an AR2G sensor (ForteBio) to activate carboxyl groups therein. Meanwhile, the 10 pg/mL recombinant human ASM protein or 2.5 pg/mL recombinant mouse ASM protein was prepared by dilution in a 10 mM sodium acetate solution (pH 5.0) (ForteBio). The solution having the diluted ASM protein was added to the AR2G sensor having activated carboxyl groups to obtain the AR2G sensor in which the recombinant human or mouse ASM protein was immobilized. A 1 M ethanol amine (ForteBio) was added to the sensor in which the obtained ASM protein was immobilized, to inactivate the remaining unreacted carboxyl groups. The antibodies produced in Example 1 were added at concentrations of 0.4, 2, or 10 nM, and the association phase of the reaction product was observed for up to about 900 seconds. Thereafter, a 1× kinetics buffer (ForteBio) was added, and the dissociation phase of the reaction product was observed for about 1,200 seconds. The association constant (Kon), dissociation constant (Kdis), and equilibrium dissociation constant (KD) of each antibody were determined using Octet^{®} analysis software (Pall Life^{®} Sciences). As a result, the analysis results for the recombinant human ASM protein are shown in Table 6, and the analysis results for the recombinant mouse ASM protein are shown in Table 7.

**TABLE 6**

| Antibody # | KD (M) | Kon (1/Ms) | Kdis (1/s) | RMax | Full R² |
|---|---|---|---|---|---|
| #9101 | 1.16E-09 | 6.26E+05 | 7.28E-04 | 0.4527 | 0.9861 |
| #9102 | 2.52E-10 | 4.16E+05 | 1.05E-04 | 0.8652 | 0.997 |
| #9104 | 2.09E-10 | 3.92E+05 | 8.18E-05 | 0.3324 | 0.9759 |
| #9108 | 1.19E-10 | 2.49E+05 | 2.96E-05 | 0.45 | 0.999 |
| #9113 | 5.64E-10 | 1.16E+06 | 6.56E-04 | 0.2868 | 0.9635 |

**TABLE 7**

| Antibody # | KD (M) | Kon (1/Ms) | Kdis (1/s) | RMax | Full R² |
|---|---|---|---|---|---|
| #9101 | 4.25E-11 | 3.76E+06 | 1.59E-04 | 0.0185 | 0.5527 |
| #9102 | 4.98E-10 | 4.89E+05 | 2.43E-04 | 0.4281 | 0.9944 |
| #9104 | 6.38E-10 | 3.10E+05 | 1.98E-04 | 0.7027 | 0.9971 |
| #9108 | 3.39E-09 | 4.59E+05 | 1.56E-03 | 0.3921 | 0.9865 |
| #9113 | <1.0E-12 | 1. 00E+06 | <1.0E-07 | 0 | 0 |

As shown in Table 6, five types of antibodies produced in Example 1 were bound to the human ASM protein with a binding affinity of 10⁻¹⁰ to 10⁻⁹ M. Meanwhile, as shown in Table 7, only the antibodies #9102, #9104, and #9108 showed a binding affinity of 10⁻¹⁰ to 10⁻⁹ M to the mouse ASM protein.

### Experimental Example 3: Identification of Antigenic Determinant of ASM Protein

The sites of the ASM protein to which the present inventive antibodies were bound, that is, antigenic determinants were investigated by hydrogen deuterium exchange mass spectrometry (HDX-MS).

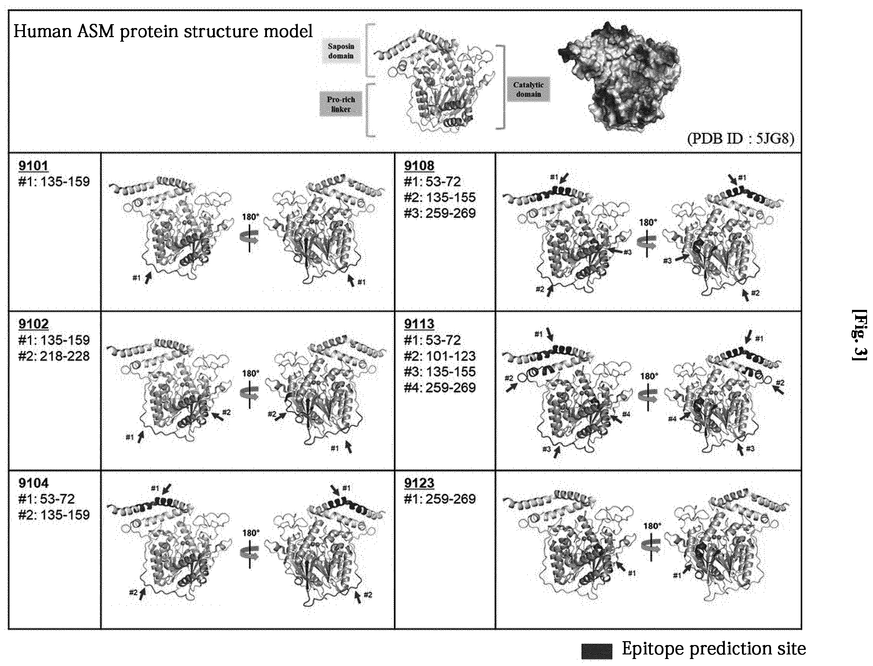

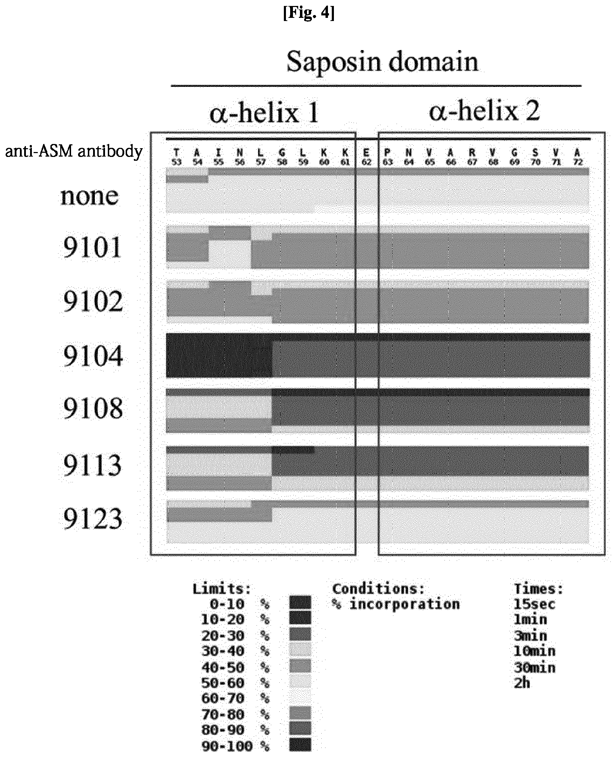

First, the 1,000 pg/mL ASM protein, 1,000 µg/mL #9101 mIgG1, 1,250 ug/mL #9102 mIgG2, 1,000 ug/mL #9104 mIgG4, 1,000 ug/mL #9108 mIgG1, 3,300 pg/mL #9113 mIgG1, or 2,932 pg/mL #9123 mIgG1 antibodies were serially diluted 2-fold to prepare up to 128-fold dilutions. Then, 1 µl of each of the prepared dilutions was taken, and mixed with an equal amount of 10 mg/ml sinapinic acid matrix (K200 MALDI kit, CovalX), and 1 µl of the mixture was dispensed in SCOUT 384 MALDI plates, followed by crystallization at room temperature. Thereafter, the crystals were measured using an MALDI mass spectrometer. For the cross-link data for non-covalent interaction analysis, 1 µl of each of the 128-fold dilutions was mixed with an equal amount of a 2 mg/ml K200 stabilizer reagent (K200 MALDI kit, CovalX), followed by incubation at room temperature for 3 hours. After the reaction was completed, 1 µl of the mixed solution was subjected to the measurement using an MALDI mass spectrometer, and the measurement results were analyzed in a high-mass MALDI MS mode. As a result, the peptide sequences of the sites that were identified to have significant difference of deuterium incorporation in the ASM protein are shown in Table 8, and human ASM protein structure models indicating the binding sites on ASM protein to each antibody are shown in FIG. 3. The deuterium exchange heat map of each antibody in the saposin domain of the ASM protein is shown in FIG. 4.

**TABLE 8**

| Antibody # | Peptide region | Amino acid sequence |
|---|---|---|
| #9101 | 135-159 | PTVPKPPPKPPSPPAPGAPVSRILF |
| #9102 | 135-159 | PTVPKPPPKPPSPPAPGAPVSRILF |
| | 218-228 | SGLGPAGPFDM |
| #9104 | 53-72 | TAINLGLKKEPNVARVGSVA |
| | 135-159 | PTVPKPPPKPPSPPAPGAPVSRILF |
| #9108 | 53-72 | TAINLGLKKEPNVARVGSVA |
| | 135-155 | PTVPKPPPKPPSPPAPGAPVS |
| | 259-269 | VRKFLGPVPVY |
| #9113 | 53-72 | TAINLGLKKEPNVARVGSVA |
| | 101-123 | VWRRSVLSPSEACGLLLGSTCGH |
| | 135-155 | PTVPKPPPKPPSPPAPGAPVS |
| | 259-269 | VRKFLGPVPVY |
| #9123 | 259-269 | VRKFLGPVPVY |

As shown in Table 8 and FIG. 3, the antibodies according to the present disclosure were bound to the fragment of amino acids at positions 53 to 72 (SEQ ID NO: 68, TAINLGLKKEPNVARVGSVA), the fragment of amino acids at positions 101 to 123 (SEQ ID NO: 69, VWRRSVLSPSEACGLLLGSTCGH), the fragment of amino acids at positions 135 to 159 (SEQ ID NO: 70, PTVPKPPPKPPSPPAPGAPVSRILF), the fragment of amino acids at positions 135 to 155 (SEQ ID NO: 71, PTVPKPPPKPPSPPAPGAPVS), the fragment of amino acid at positions 218 to 228 (SEQ ID NO: 72, SGLGPAGPFDM), or the fragment of amino acids at positions 259 to 269 (SEQ ID NO: 73, VRKFLGPVPVY) from the N-terminus of the ASM protein. Especially, as shown in FIG. 4, the antibodies according to the present disclosure were mainly bound to α-helix 1 and α-helix 2 in the saposin domain.

### Experimental Example 4: Inhibition of ASM protein activity

### 4-1. Inhibition of ASM protein activity

It was examined whether the produced antibodies, specifically binding to the ASM protein, inhibited the activity of the ASM protein located in the cellular membrane.

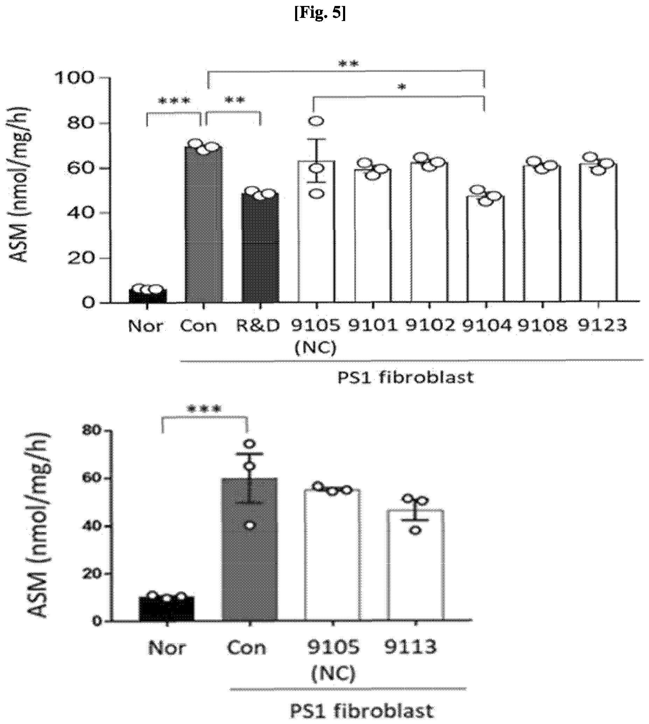

First, fibroblasts (Coriell Institute) of Alzheimer's patients were treated with the antibodies produced in Example 1 at a concentration of 3 pg/ml, and incubated at 37°C for 24 hours. Thereafter, the cells were collected, and the cell membrane was separated using the Mem-PER^{™} Plus Membrane Protein Extraction Kit (Thermo) according to the manufacturer's protocol, followed by protein extraction. The extracted protein as a sample was placed into an insert for UPLC and subjected to UPLC analysis (Waters, 186004800) by a common method, and the results are shown in FIG. 5. The antibody #9105 confirmed not to bind to the ASM protein was used as a control group.

As shown in FIG. 5, the antibody #9104 among the six types of antibodies produced in Example 1 significantly inhibited the activity of the ASM protein increased in the cellular membrane of Alzheimer's patients.

### 4-2. Inhibition of ASM protein activity according to treatment concentration of anti-ASM antibody

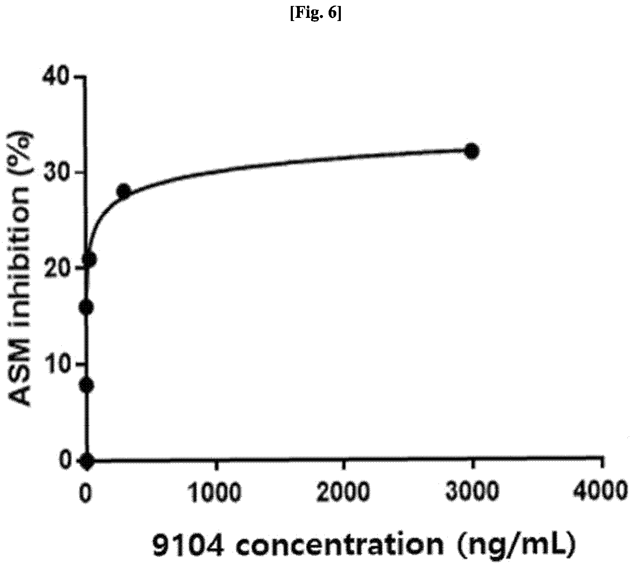

The change in activity of the ASM protein located in the cellular membrane was examined by the same method as in Experimental Example 4-1 except for the treatment with antibody #9104 produced in Example 1 at a concentration of 0.03, 0.3, 3, 30, 300, or 3,000 ng/ml. As a result, the analyzed ASM protein activity inhibition (%) is shown in FIG. 6 and Table 9. Antibody #9105 was used as a control group.

**TABLE 9**

| Treatment concentration (ng/ml) | ASM protein activity inhibition (%) | |
|---|---|---|
| | #9104 | #9105 |
| 0.03 | 0 | - |
| 0.3 | 7.9 | - |
| 3 | 16 | - |
| 30 | 21 | - |
| 300 | 28 | - |
| 3,000 | 32 | 9 |

As shown in FIG. 6 and Table 9, antibody #9104 according to the present disclosure significantly inhibited the activity of the ASM protein located in the cellular membrane depending on the treatment concentration. However, antibody #9105 used as a control group slightly inhibited the activity of the ASM protein located in the cellular membrane only at a treatment concentration of 3,000 ng/ml or more.

### Experimental Example 5: Sphingolipid metabolism alteration

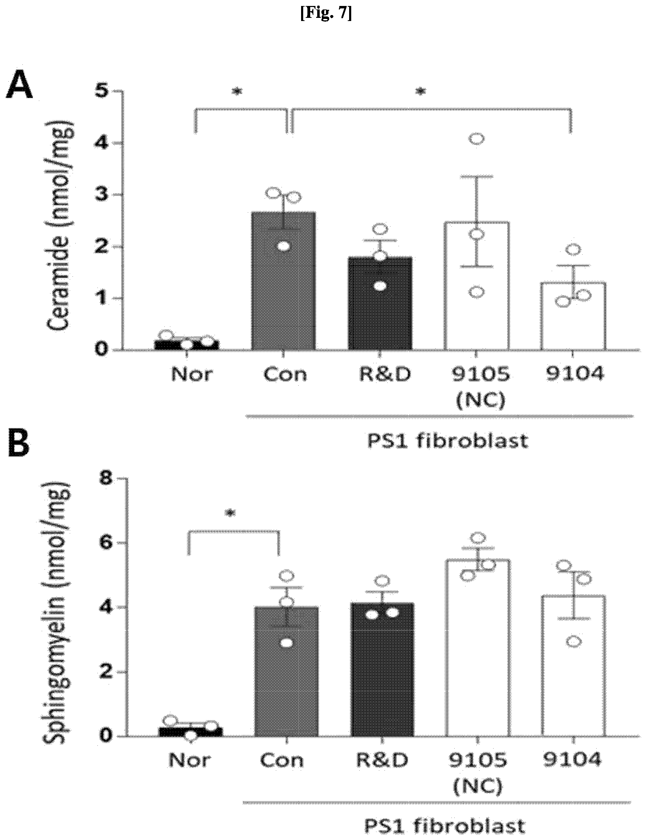

The alteration of sphingolipid metabolism in the cellular membrane of the cells treated with antibody #9104 at 3 µg/ml as described in Experimental Example 4-1 was examined through changes in sphingomyelin and ceramide contents. The experiment was performed by a usual method, and the measured sphingomyelin and ceramide contents are shown in FIG. 7.

As shown in FIG. 7, the content of ceramide present in the cellular membrane of fibroblasts of Alzheimer's patients was significantly reduced by the treatment with antibody #9104, but there was no significant change in content of sphingomyelin.

The result indicated that the antibody according to the present disclosure inhibited the decomposition of sphingomyelin by ASM protein.

### Experimental Example 6: Cognitive memory improvement

The change in cognitive memory of mice was examined by administering antibody #9104 to APP/PS1 mice, Alzheimer's disease animal models.

### 6-1. Administration of anti-ASM antibody

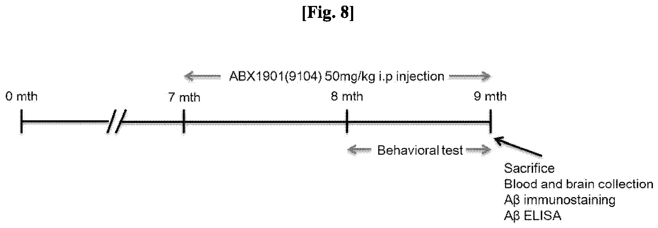

APP/PS1 mice aged 7 months were intraperitoneally administered with 324 µl of antibody #9104 at a dose of 50 mg/kg for 8 weeks. The administration was performed twice a week (with an interval of 3 days), and antibody #9104 was administered a total of 16 times (FIG. 8). The descendant mice born from the crossbreeding of C57BL/6 and APP/PS1 mice and not treated with any substance were used for a non-treatment control group, and APP/PS1 mice treated with 324 µl of PBS were used for a negative control group.

### 6-2. Cognitive memory improvement-(1)

A behavioral test was conducted from the 41st day to the last day of administration of antibody #9104 to examine a change in cognitive memory by the administration of the antibody.

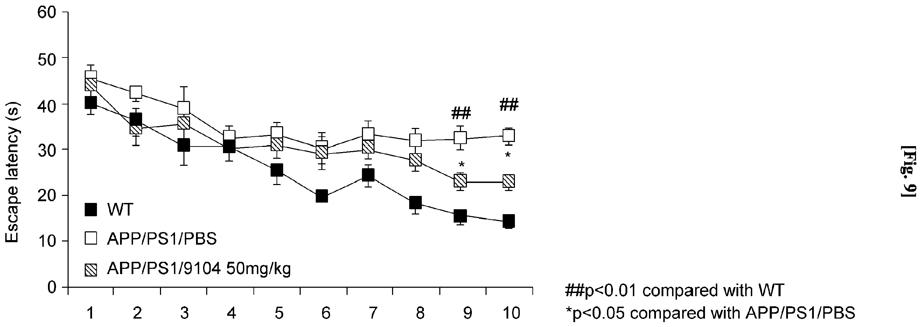

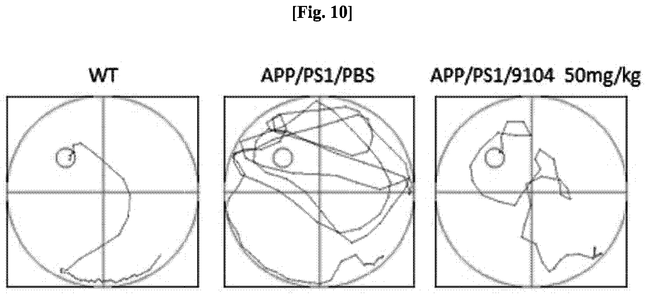

First, the repulsion of each mouse against a tester was eliminated by repeatedly holding the tail of the mouse and placing the mouse on the arm and the back of the hand of the tester for 5 minutes from the 41st day of administration, and thereafter, the repulsion of the mouse against water was eliminated by acclimating the mouse to water for 30 minutes, before the test. A platform was installed while cues were removed from four sides of a water tank, and a small triangular water tank was installed based on the platform. The mouse was placed in the water tank and trained to swim and climb onto the platform 10 times. The mouse was allowed to stay for 10 seconds whenever the mouse climbed onto the platform 10 seconds after swim training, and after 10 seconds, the mouse was allowed to re-enter the water. This was repeated 10 times, during which the mouse was trained to climb onto the platform in various directions while the direction of approach to the platform was changed. A Morris water maze test was conducted from the next day, the 42nd day to the 51st day of administration. Specifically, cues were placed on the four sides of the water tank, and each mouse was tested four times (a time limit of 60 seconds for each time) at the same time every day, and this was repeated a total of 10 rounds to record the time required to climb onto the platform. On the 52nd day of administration, a probe test was conducted to record the existing location where the platform has been present in the water tank with the platform removed, and the path length and speed of swimming past other quadrants. As a result, the Morris water maze test results are shown in FIG. 9, and the probe test results are shown in FIGS. 10 to 12.

As shown in FIG. 9, as the test was repeated, the escape latency time of the mice administered with antibody #9104 was significantly reduced. The mice of the negative control group showed an escape latency time of 30 seconds on average, indicating the impairment of cognitive function, and the mice of the non-treatment control group showed a reduction in escape latency time with the repetition of the test. Specifically, the group administered with antibody #9104 showed a 31.5% reduction in escape latency time in the 9th round and a 30.8% reduction in the 10th round, compared with the mice of the negative control group.

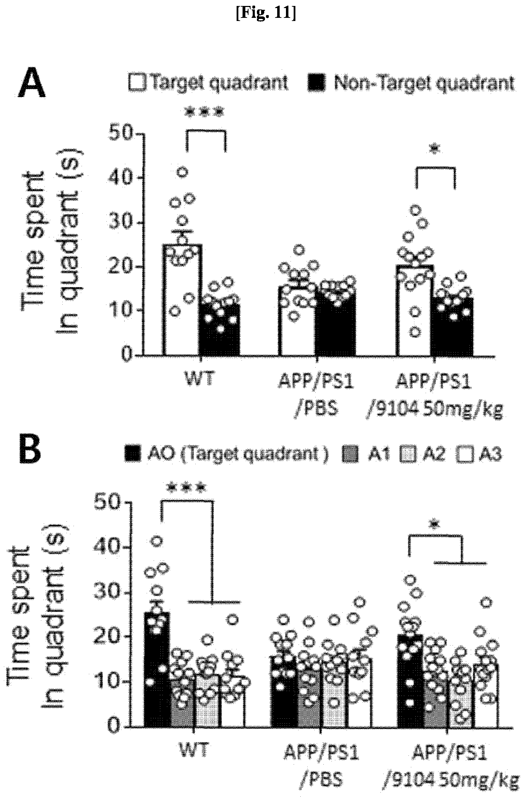

As shown in FIGS. 10 and 11, the mice administered with antibody #9104 showed the time spent in the target quadrant significantly longer than the time spent in the non-target quadrants. This result was similar to that of the non-treatment control group, and was differentiated from that of the negative control group showing little difference between the time spent in the target quadrant and the time spent in the non-target quadrants.

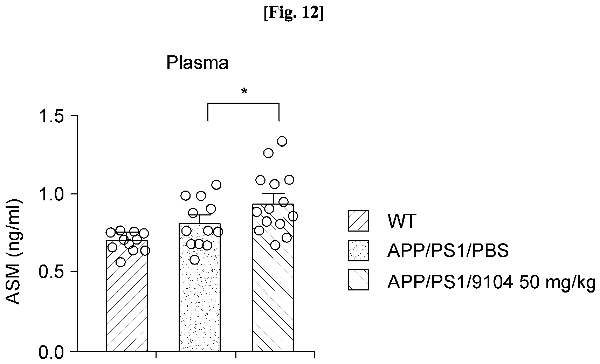

As shown in FIG. 12C, the frequency of crossing platform was significantly increased in the group administered with antibody #9104 but decreased in the negative control group. As shown in FIGS. 12A and 12B\, all the mouse groups were similar in view of path length and swim speed, indicating that the administration of antibody #9104 did not affect exercise ability.

### 6-3. Cognitive memory improvement-(2)

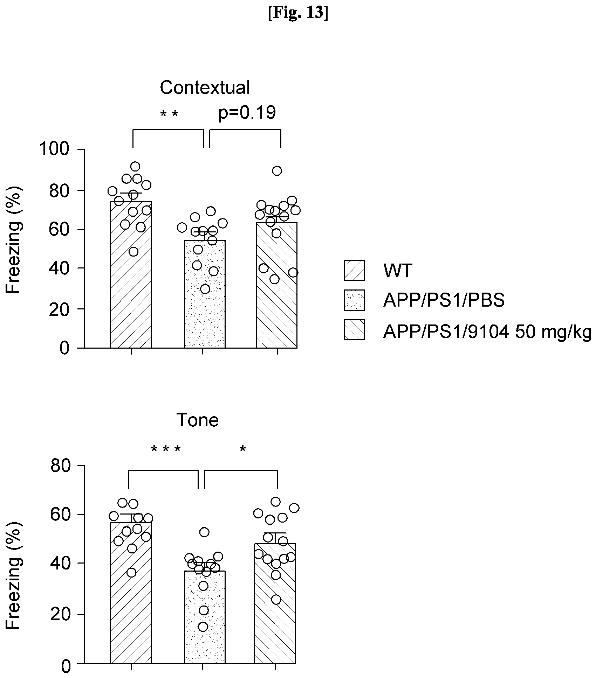

The change in cognitive memory of mice was examined by administering antibody #9104 at a dose of 50 mg/kg to APP/PS1 mice, Alzheimer's disease animal models. Therefore, antibody #9104 was administered by the same method as in Experimental Example 6-1 and a contextual and cued fear conditioning test was conducted by a common method, and then the results are shown in FIG. 13.

As shown in FIG. 13, the mice administered with antibody #9104 showed a significant increase in freezing according to the contextual or tone condition, but the mice of the negative control group showed a reduction in freezing.

The results indicated that the antibody of the present disclosure significantly improved the cognitive memory in Alzheimer's disease animal models.

### Experimental Example 7: ASM protein activity

### 7-1. ASM protein activity

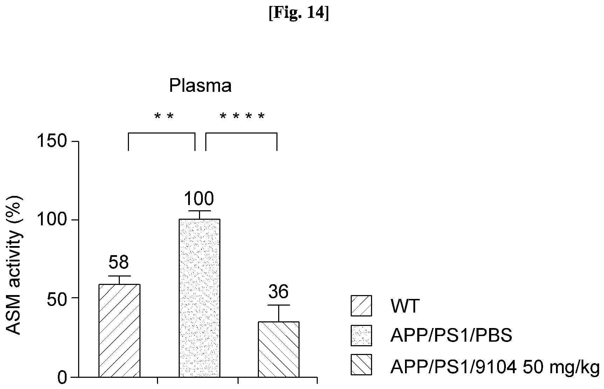

The activity of the ASM protein present in the blood and brain tissue of the animal models confirmed to have improved cognitive memory in Experimental Example 6 was examined. Specifically, proteins were obtained from the blood plasma of the mice by a common method, and the obtained proteins were used as a sample and subjected to UPLC by the same method as in Experimental Example 4-1. As a result, the results obtained by calculating the ASM activity (%) of the mice of the non-treatment control group and the mouse group administered with #9104 relative to the activity of the ASM protein of the mice of the negative control group are shown in FIG. 14.

As shown in FIG, 14, the activity of the ASM protein present in the blood plasma was inhibited by about 64% by administration of #9104.

### 7-2. ASM protein level

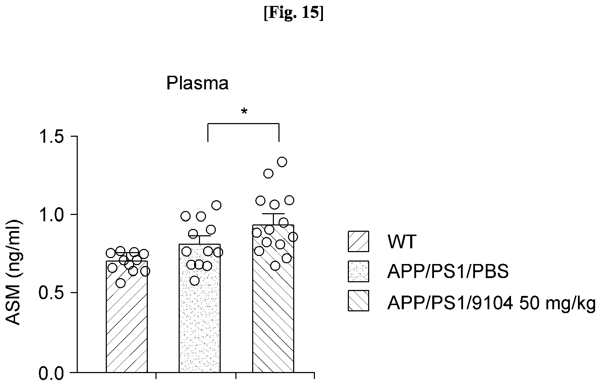

ELISA analysis was performed to examine whether the inhibition of ASM protein activity confirmed in Experimental Example 7-1 was due to a reduction in expression level of the protein. The blood plasma tissue obtained in Experimental Example 7-1 was used as a sample, and the analysis was performed using the mouse ASM ELISA kit (Mybiosource) according to the manufacturer's protocol. The results are shown in FIG. 15.

As shown in FIG. 15, the ASM protein level was significantly increased in the mice administered with antibody #9104 rather than the non-treatment control group or the negative control group.

The results indicated that the antibody according to the present disclosure inhibited the activity of the ASM protein without affecting the ASM protein level.

### Experimental Example 8: Inhibition of amyloid-β(Aβ) accumulation

The accumulation of amyloid-β present in the brain tissue of the animal models confirmed to have improved cognitive memory in Experimental Example 6 was examined by thioflavin-S staining, western blotting using 6E10 antibody, and ELISA.

### 8-1. Inhibition of Aβ accumulation-(1)

The animal models of Experimental Example 6 were anesthetized with 2.5% avertin, and the chest cavity was opened to obtain the blood from the heart by using a 1-cc syringe. After the blood was obtained, perfusion was performed with 20 ml of PBS, and additional perfusion was performed with 20-30 ml of 4% paraformaldehyde (PFA). The mouse brain was excised, and left overnight while placed in a 4% PFA solution, and then the tissue was cut into a 30-um thick slices by a vibratome. The obtained brain tissue slices were placed in a 12-well plate, and then 1 ml of a PBS solution was added, followed by stirring at 33 rpm for 5 minutes at room temperature. The PBS solution was removed, and Aβ plaques were stained for 10 minutes by treatment with 500 µℓ of a 0.5% thioflavin-S reagent diluted in ethanol. Thereafter, the slices were washed two times with 50% ethanol and washed once with 1 ml of a PBS solution. The slices were mounted by addition of mount media containing DAPI.

For staining of all types of Aβ proteins, the brain tissue slices were placed in a 12-well plate, and then 1 ml of a PBS solution was added, followed by stirring at 33 rpm for 5 minutes at room temperature, and then the PBS solution was removed. Thereafter, 1 ml of a PBS solution containing 0.2% Triton X-100 was added thereto, and stirred at 33 rpm for 1 hour. After stirring, the PBS solution containing Triton X-100 was removed, and the slices were pre-treated with a PBS solution containing 0.05% Triton X-100 for 1 hour. A mixture of 6E10 antibody (Biolegend) and a PBS solution containing 0.05% Triton X-100 at a volume ratio of 1:1 was added at an amount 500 µl of to the pre-treated brain tissue slices. The slices were incubated at 4°C overnight with stirring, and washed three times with a PBS solution containing Triton X-100. The slices were treated with a mouse-488 secondary antibody, incubated at room temperature for 2 hours, and then washed three times with a PBS solution containing Triton X-100. The slices were mounted by addition of mount media containing DAPI.

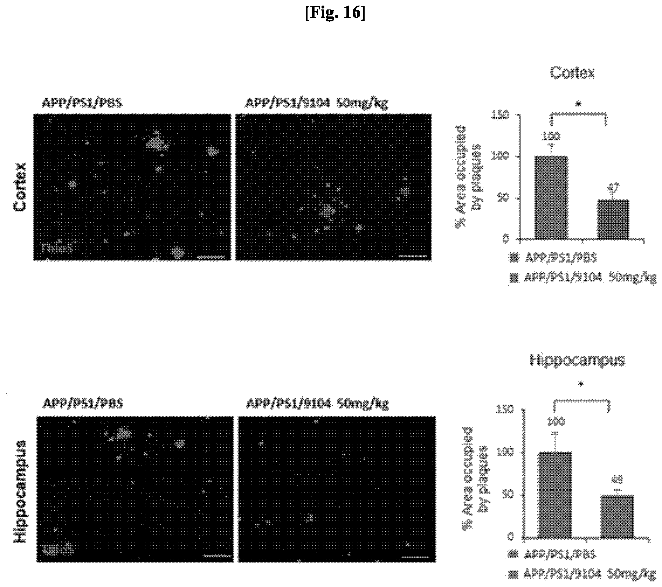

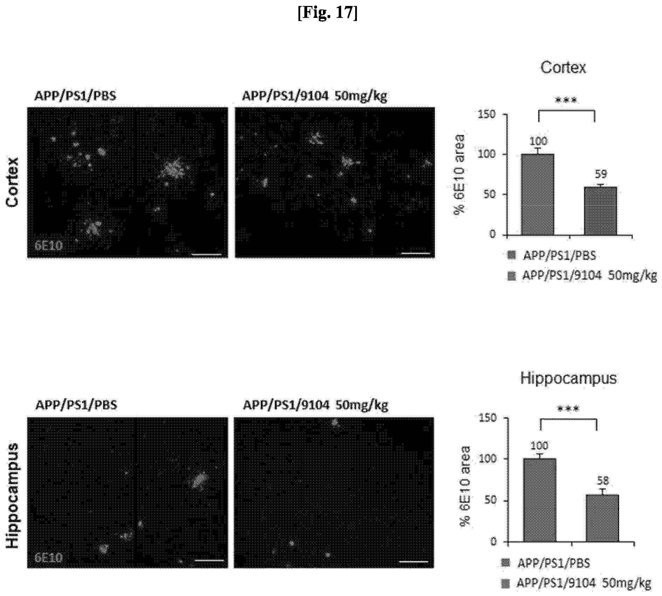

The stained brain tissue slices were imaged at a magnification of 20 times by using a laser-scanning confocal microscope (FV3000, Olympus), and the stained area was analyzed by using MetaMorph software (Molecular Devices). The analysis results are shown in FIGS. 16 and 17.

As shown in FIGS. 16 and 17, dense-core and diffuse amyloid-β plaques present in the cortex and hippocampus of the brain were significantly reduced by the treatment with antibody #9104.

### 8-2. Inhibition of Aβ accumulation-(2)

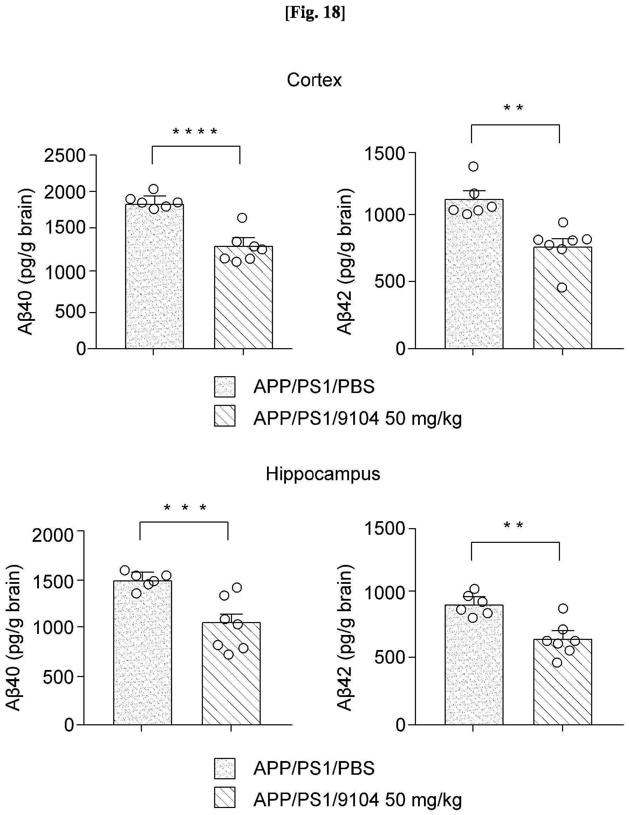

The obtained brain tissue was separated into the cortex and the hippocampus, and RIPA buffer containing 100 mM PMSF and 1% protease inhibitor was added thereto, followed by tissue homogenization. The homogenized tissues were centrifuged for 10 minutes under conditions of 4°C and 13,000 rpm, to obtain supernatants. The obtained supernatants were further centrifuged for 30 minutes under the same conditions, to obtain supernatants again, and these supernatants were used as samples to measure the levels of Aβ therein by using an amyloid-β40 ELISA kit (KHB3481, Invitrogen) and an amyloid-β42 ELISA kit (KHB3441, Invitrogen). The obtained results are shown FIG. 18.

As shown in FIG. 18, Aβ40 present in the cortex and hippocampus of the brain was reduced to 30.9% and 30.9%, respectively, by antibody #9104, and these reductions were significant compared with the negative control group. In addition, Aβ42 was significantly reduced by the administration of antibody #9104 compared with the negative control group.

### Experimental Example 9: Tau protein accumulation

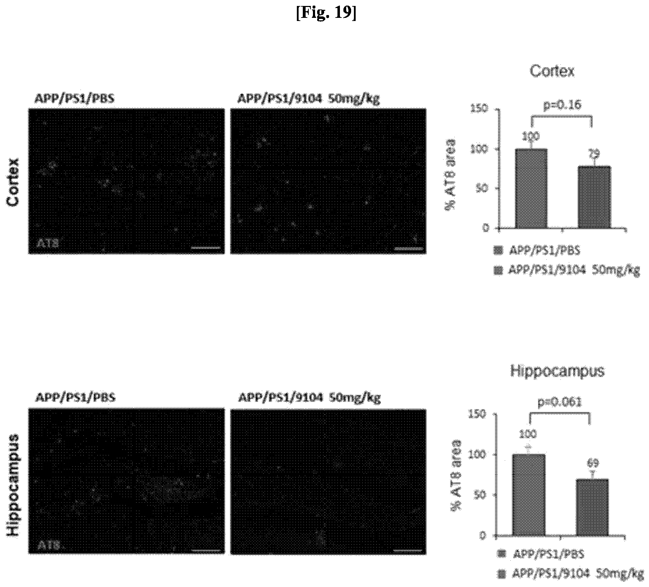

The brain tissue slices obtained in Experimental Example 8 were used to examine the change in tau accumulation by staining the tau protein. Specifically, the experiment was conducted under the same conditions and method as in Experimental Example 8-1 except that anti-AT8 antibody (ThermoFisher Scientific, MN1020) was used as a primary antibody. The analysis results are shown in FIG. 19.

As shown in FIG 19, the tau protein present in the cortex and hippocampus of the brain was significantly reduced by the treatment with antibody #9104.

### Experimental Example 10: Alleviation of neuroinflammation

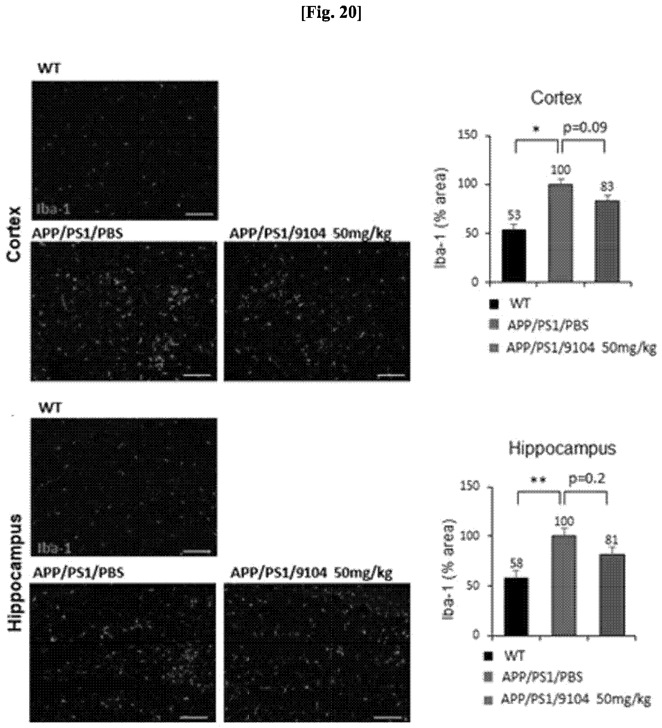

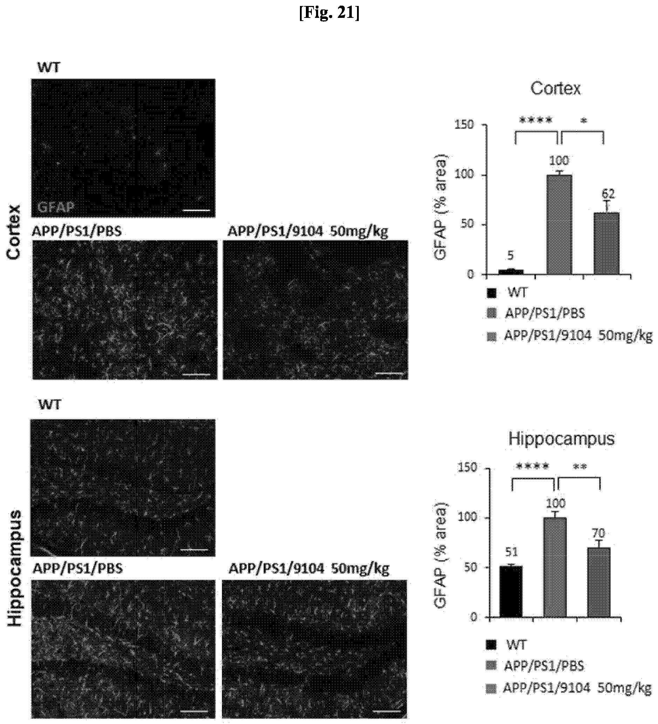

The neuroinflammation alleviating effect by antibody #9104 in the brain tissue of the animal models confirmed to have improved cognitive memory in Experimental Example 6 was examined through activation of microglia and astrocytes. The experiment was conducted under the same conditions and method as in Experimental Example 8-1 except that anti-lba-1 antibody (Wako, 019-19941) or anti-GFAP antibody (Dako, N1506) was used as a primary antibody, and rabbit-488 secondary antibody was used as a secondary antibody. The analysis results are shown in FIGS. 20 and 21.

As shown in FIGS. 20 and 21, the expressions of lba-1 and GFAP proteins present in the cortex and hippocampus of the brain were significantly reduced, and thus the activity of microglia and astrocytes in the brain tissue was inhibited by #9104 antibody.

The results indicated that the antibody according to the present disclosure had an effect of alleviating brain neuroinflammation.

### Experimental Example 11: Toxicity

The toxicity of antibody #9104 was examined by the following method.

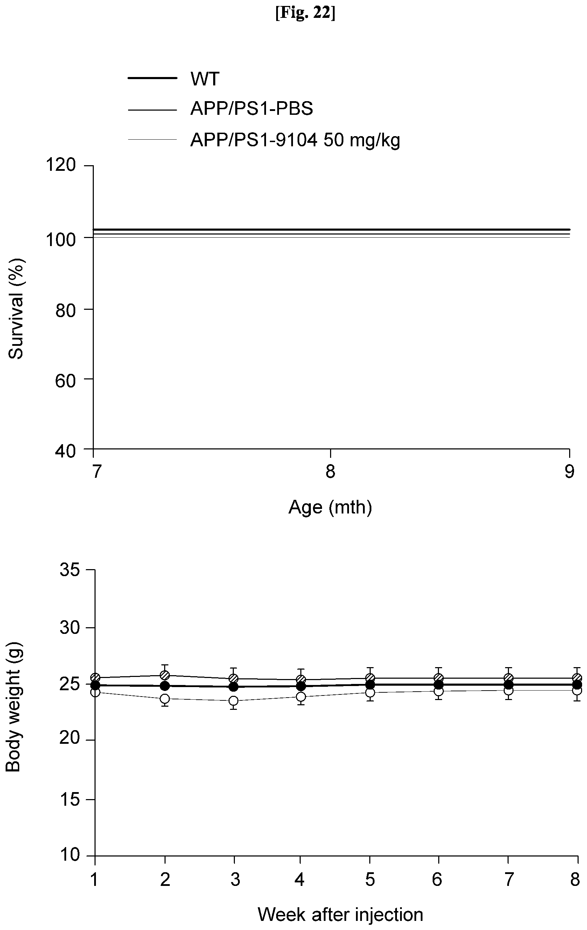

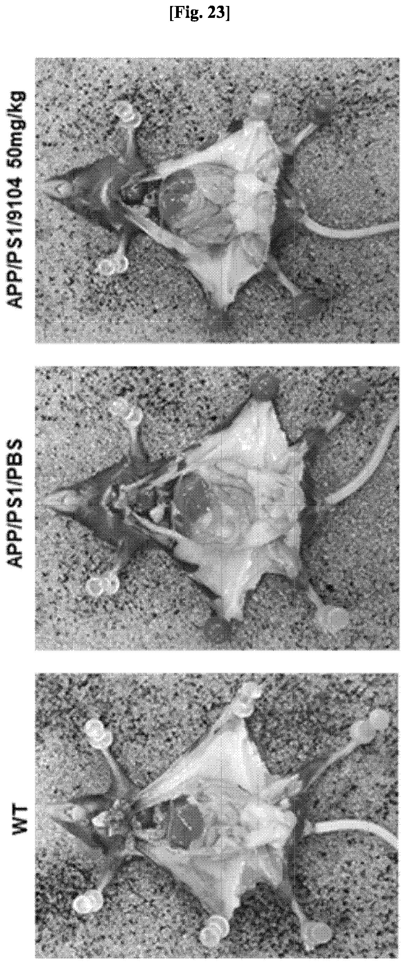

The experiment was conducted while antibody #9104 was administered in Experimental Example 6. Specifically, the survival rate was assessed by visually observing the movement and abnormal signs of mice twice a week, and the mice were weighed on the same day and time once a week. The mice after completion of the administration were necropsied by a common method, and organs were observed to determine the presence or absence of organ toxicity due to #9104 antibody. As a result, the changes in survival rate and body weight of the mice are shown in FIG. 22, and the observation results of organ toxicity are shown in FIG. 23.

As shown in FIGS. 22 and 23, there were no changes in survival rate and body weight by the treatment with antibody #9104 antibody, and the organs were also confirmed to be normal.

The results indicated that the antibody according to the present disclosure can be significantly used in the treatment of a brain disease, such as Alzheimer's disease, even without toxicity.

## Claims

1. A pharmaceutical composition for preventing or treating a brain disease, wherein the pharmaceutical composition comprises as an active ingredient an antibody or antigen-binding fragment thereof that specifically binds to an acid sphingomyelinase (ASM) protein.

2. The pharmaceutical composition of claim 1, wherein the antibody or antigen-binding fragment thereof binds to at least one epitope selected from the group consisting of a fragment of amino acids at positions 53 to 72, a fragment of amino acids 101 to 123, a fragment of amino acids 135 to 159, a fragment of amino acids 135 to 155, a fragment of amino acids 218 to 228, and a fragment of amino acids 259 to 269 from the N-terminus of the ASM protein.

3. The pharmaceutical composition of claim 1, wherein the antibody or antigen-binding fragment thereof binds to at least one epitope selected from the group consisting of polypeptides set forth in SEQ ID NOS: 68 to 73, respectively.

4. The pharmaceutical composition of claim 1, wherein the antibody or antigen-binding fragment thereof comprises:
a heavy chain variable region comprising a heavy chain CDR1 (X₁YX₂MS) consisting of the amino acid sequence set forth in SEQ ID NO: 61, a heavy chain CDR2 (X₃IX₄X₅X₆X₇X₈X₉X₁₀YYADSVKG) consisting of the amino acid sequence set forth in SEQ ID NO: 62, and a heavy chain CDR3 selected from the group consisting of amino acid sequences set forth in SEQ ID NOS: 27, 30, 33, 36, 39, and 42, respectively; and
a light chain variable region comprising a light chain CDR1 (X₁₁GSSSNIGX₁₂NX₁₃VX₁₄) consisting of the amino acid sequence set forth in SEQ ID NO: 63, a light chain CDR2 (X₁₅X₁₆X₁₇X₁₈RPS) consisting of the amino acid sequence set forth in SEQ ID NO: 64, and a light chain CDR3 (X₁₉X₂₀WDX₂₁SLX₂₂X₂₃YV) having the amino acid sequence set forth in SEQ ID NO 65.

5. The pharmaceutical composition of claim 4, wherein:
X₁ and X₂ each are selected from the group consisting of asparagine (Asn), glycine (Gly), serine (Ser), aspartic acid (Asp), alanine (Ala), and tyrosine (Tyr);
X₃ to X₁₀ each are selected from the group consisting of glycine, leucine (Leu), alanine, serine, tyrosine, proline (Pro), asparagine, lysine (Lys), and isoleucine (Ile);
X₁₁ to X₁₄ each are selected from the group consisting of threonine (Thr), serine, asparagine, alanine, proline, and tyrosine;
X₁₅ to X₁₈ each are selected from the group consisting of tyrosine, alanine, aspartic acid, serine, asparagine, histidine (His), glutamine (Gln), and lysine; and
X₁₉ to X₂₃ each are selected from the group consisting of glycine, alanine, serine, threonine, tyrosine, aspartic acid, and asparagine.

6. The pharmaceutical composition of claim 5, wherein:
X₁ is asparagine, glycine, serine, or aspartic acid;
X₂ is alanine or tyrosine;
X₃ is glycine, leucine, alanine, or serine;
X₄ is tyrosine or serine;
X₃ is proline or tyrosine;
X₆ is asparagine or glycine;
X₇ and X₈ each are glycine or serine;
X₉ is asparagine or serine;
X₁₀ is lysine or isoleucine;
X₁₁ is threonine or serine;
X₁₂ is asparagine or serine;
X₁₃ is alanine, proline, threonine, or tyrosine;
X₁₄ is asparagine, tyrosine, or serine;
X₁₅ is tyrosine, alanine, aspartic acid, or serine;
X₁₆ is aspartic acid or asparagine;
X₁₇ is serine or asparagine;
X₁₈ is histidine, glutamine, or lysine;
X₁₉ is glycine or alanine;
X₂₀ is alanine, serine, or threonine;
X₂₁ is tyrosine, serine, alanine, or aspartic acid;
X₂₂ is serine or asparagine; and
X₂₃ is alanine or glycine.

7. The pharmaceutical composition of claim 1, wherein the antibody or antigen-binding fragment thereof comprises:
a heavy chain variable region comprising,
a heavy chain CDR1 selected from the group consisting of an amino acid sequence set forth in SEQ ID NOS: 25, 28, 31, 34, 37, and 40, respectively,
a heavy chain CDR2 selected from the group consisting of amino acid sequences set forth in SEQ ID NOS: 26, 29, 32, 35, 38, and 41, respectively, and
a heavy chain CDR3 selected from the group consisting of amino acid sequences set forth in SEQ ID NOS: 27, 30, 33, 36, 39, and 42, respectively; and
a light chain variable region comprising,
a light chain CDR1 selected from the group consisting of amino acid sequences set forth in SEQ ID NOS: 43, 46, 49, 52, 55, and 58, respectively,
a light chain CDR2 selected from the group consisting of amino acid sequences set forth in SEQ ID NOS: 44, 47, 50, 53, 56, and 59, respectively, and
a light chain CDR3 selected from the group consisting of amino acid sequences set forth in SEQ ID NOS: 45, 48, 51, 54, 57, and 60, respectively.

8. The pharmaceutical composition of claim 1, wherein the antibody or antigen-binding fragment thereof comprises:
a heavy chain variable region selected from the group consisting of,
a heavy chain variable region comprising heavy chain CDR1, CDR2, and CDR3 consisting of the amino acid sequences set forth in SEQ ID NOS: 25, 26, and 27, respectively,
a heavy chain variable region comprising heavy chain CDR1, CDR2, and CDR3 consisting of the amino acid sequences set forth in SEQ ID NOS: 28, 29, and 30, respectively,
a heavy chain variable region comprising heavy chain CDR1, CDR2, and CDR3 consisting of the amino acid sequences set forth in SEQ ID NOS: 31, 32, and 33, respectively,
a heavy chain variable region comprising heavy chain CDR1, CDR2, and CDR3 consisting of the amino acid sequences set forth in SEQ ID NOS: 34, 35, and 36, respectively,
a heavy chain variable region comprising heavy chain CDR1, CDR2, and CDR3 consisting of the amino acid sequences set forth in SEQ ID NOS: 37, 38, and 39, respectively, and
a heavy chain variable region comprising heavy chain CDR1, CDR2, and CDR3 consisting of the amino acid sequences set forth in SEQ ID NOS: 40, 41, and 42, respectively; and
a light chain variable region selected from the group consisting of,
a light chain variable region comprising light chain CDR1, CDR2, and CDR3 consisting of the amino acid sequences set forth in SEQ ID NOS: 43, 44, and 45, respectively,
a light chain variable region comprising light chain CDR1, CDR2, and CDR3 consisting of the amino acid sequences set forth in SEQ ID NOS: 46, 47, and 48, respectively,
a light chain variable region comprising light chain CDR1, CDR2, and CDR3 consisting of the amino acid sequences set forth in SEQ ID NOS: 49, 50, and 51, respectively,
a light chain variable region comprising light chain CDR1, CDR2, and CDR3 consisting of the amino acid sequences set forth in SEQ ID NOS: 52, 53, and 54, respectively,
a light chain variable region comprising light chain CDR1, CDR2, and CDR3 consisting of the amino acid sequences set forth in SEQ ID NOS: 55, 56, and 57, respectively, and
a light chain variable region comprising light chain CDR1, CDR2, and CDR3 consisting of the amino acid sequences set forth in SEQ ID NOS: 58, 59, and 60, respectively.

9. The pharmaceutical composition of claim 1, wherein the antibody or antigen-binding fragment thereof comprises:
a heavy chain variable region comprising heavy chain CDR1, CDR2, and CDR3 consisting of the amino acid sequences set forth in SEQ ID NOS: 25, 26, and 27, respectively, and a light chain variable region comprising light chain CDR1, CDR2, and CDR3 consisting of the amino acid sequences set forth in SEQ ID NOS: 43, 44, and 45, respectively;
a heavy chain variable region comprising heavy chain CDR1, CDR2, and CDR3 consisting of the amino acid sequences set forth in SEQ ID NOS: 28, 29, and 30, respectively, and a light chain variable region comprising light chain CDR1, CDR2, and CDR3 consisting of the amino acid sequences set forth in SEQ ID NOS: 46, 47, and 48, respectively;
a heavy chain variable region comprising heavy chain CDR1, CDR2, and CDR3 consisting of the amino acid sequences set forth in SEQ ID NOS: 31, 32, and 33, respectively, and a light chain variable region comprising light chain CDR1, CDR2, and CDR3 consisting of the amino acid sequences set forth in SEQ ID NOS: 49, 50, and 51, respectively;
a heavy chain variable region comprising heavy chain CDR1, CDR2, and CDR3 consisting of the amino acid sequences set forth in SEQ ID NOS: 34, 35, and 36, respectively, and a light chain variable region comprising light chain CDR1, CDR2, and CDR3 consisting of the amino acid sequences set forth in SEQ ID NOS: 52, 53, and 54, respectively;
a heavy chain variable region consisting of heavy chain CDR1, CDR2, and CDR3 consisting of the amino acid sequences set forth in SEQ ID NOS: 37, 38, and 39, respectively, and a light chain variable region consisting of light chain CDR1, CDR2, and CDR3 consisting of the amino acid sequences set forth in SEQ ID NOS: 55, 56, and 57, respectively; or
a heavy chain variable region consisting of heavy chain CDR1, CDR2, and CDR3 consisting of the amino acid sequences set forth in SEQ ID NOS: 40, 41, and 42, respectively, and a light chain variable region consisting of light chain CDR1, CDR2, and CDR3 consisting of the amino acid sequences set forth in SEQ ID NOS: 58, 59, and 60, respectively.

10. The pharmaceutical composition of claim 1, wherein the ASM protein is derived from a mammal.

11. The pharmaceutical composition of claim 1, wherein the ASM protein is a polypeptide consisting of an amino acid sequence set forth in SEQ ID NO: 66 or 67.

12. The pharmaceutical composition of claim 1, wherein the brain disease is a degenerative brain disease.

13. The pharmaceutical composition of claim 12, wherein in the degenerative brain disease, the expression or aggregation level of amyloid-β is higher or at risk of being higher than normal.

14. The pharmaceutical composition of claim 12, wherein the degenerative brain disease is dementia, Alzheimer's disease, Parkinson's disease, Huntington's disease, mild cognitive impairment, cerebral amyloid angiopathy, Down syndrome, amyloid stroke, systemic amyloid disease, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, or frontotemporal dementia.

15. A health functional food for preventing or alleviating a brain disease, the health functional food comprising as an active ingredient an antibody or antigen-binding fragment thereof that specifically binds to an ASM protein.

16. A method for preventing, alleviating, or treating a brain disease, the method including a step of administering to a subject an antibody or antigen-binding fragment thereof that specifically binds to an ASM protein.

17. An antibody or antigen-binding fragment thereof that specifically binds to an ASM protein for use in a method for preventing, alleviating, or treating a brain disease.
